# EUROPEAN PATENT SPECIFICATION

(11) **EP 0 946 753 B1**
(45) Date of publication and mention of the grant of the patent: **09.06.2004**
(21) Application number: 97953011.0
(22) Date of filing: 16.12.1997
(51) Int. Cl.: C12Q 1/68

(54) **PHARMACOGENETIC METHODS FOR USE IN THE TREATMENT OF NERVOUS SYSTEM DISEASES**
PHARMAZEUTISCH-GENETISCHE VERFAHREN ZUR VERWENDUNG BEI DER BEHANDLUNG VON KRANKHEITEN DES NERVENSYSTEMS
PROCEDES PHARMACOGENETIQUES DESTINES AU TRAITEMENT DE MALADIES DU SYSTEME NERVEUX

(30) Priority: 16.12.1996 US 766975
(43) Date of publication of application: 06.10.1999
(73) Proprietor: McGill University, Montreal, Quebec H3A 2T6 (CA); Nova Molecular, Inc., Montreal, Quebec H4A 3S5 (CA)
(72) Inventor: POIRIER, Judes, Boisbriand, Quebec J7G 2T9 (CA); WIEBUSCH, Heiko, Montreal, Quebec H3S 2E8 (CA); SCHAPPERT, Keith, Montreal, Quebec H2X 3R2 (CA)
(74) Representative: Vossius, Volker, Dr.
(86) International application number: PCT/IB1997/001648
(87) International publication number: WO 1998/027227

(56) References cited:
- WO-A-94/09155
- WO-A-95/29257
- WO-A-96/02670
- WO-A-96/03656
- SORBI ET AL.: "ApoE as a diagnostic factor for post-traumatic coma" NATURE MEDICINE, vol. 1, no. 9, September 1995, page 852 XP002062261

## Description

### BACKGROUND OF THE INVENTION

The invention relates to methods for the treatment of neurological disease.

Apolipoprotein E (apoE) functions as a ligand in the process of receptor mediated internalization of lipid-rich lipoproteins. ApoE is probably also involved in reverse lipid transport. In the central nervous system (CNS), apoE plays a central role in the mobilization and redistribution of cholesterol and phospholipid during membrane remodeling associated with synaptic plasticity (Pobier J. et al., 1991, *Mol. Brain.* Res., 9:191-195; Poirier J. et al., 1991, *Mol. Brain.* Res., 11:97-106; Poirier J. et al., 1993, *Neuroscience,* 55:81-90). The importance of apoE in the brain is further underscored by the absence of other key plasma apolipoproteins such as apoA1 and apoE in the brain (Roheim P.S. et al., 1979, *Proc. Natl*. *Acad Sci.,* 76:4646-4649).

The apoE gene on chromosome 19 has three common alleles (E2, E3, E4), which encode three major apoE isoforms. The frequency of the apoE4 allele has been shown to be markedly increased in sporadic Alzheimer's Disease (AD) (Poirier J. et al., 1993, *Lancet,* 342:697-699; Noguchi S. et al., 1993, *Lancet* (letter), 342:737) and late onset familial Alzheimer's disease (AD) (Corder E.H. et al., 1993, Science, 261:921-923; Payami H. et aL, 1993, *Lancet* (letter), 342:738). An apoE gene dosage effect was observed in both sporadic and familial cases (i.e., as age of onset increases, E4 allele copy number decreases). Women, who are generally at a greater risk of developing Alzheimer's disease, showed an increase in apoE4 allele freqnency when compared to age matched men.

WO 95/29257 teaches methods for determining the clinical responsiveness of subjects with a neurodegenerative disease, such as Alzheimer's disease, to suitable therapeutics.

WO 96/02670 teaches a method of prognosing the disease progression and ultimate degree of dementia that a subject would suffer during a beta amyloid-related disease by detecting the absence or presence of ApoE isoforms or DNA

WO 96/03656 teaches a method of prognosing the likelihood of the development of chronic neurodegenerative pathology in a head-injured subject, or a subject at risk of head injury, by detecting ApoE isoforms in the subject.

The cholinergic hypothesis of geriatric memory dysfunction raised some fundamental questions regarding the heterogeneity of responses toward different cholinomimetics in AD (Bartus R.T. et al., 1982, *Science,* 217:408-417). The absence of clear beneficial effects of choline and lecithin on geriatric patients with and without AD is still perplexing. Furthermore, multiple clinical studies using esterase inhibitors such as physostigmine and tacrine have shown that contrary to young subjects, the optimal acute dose necessary to facilitate performance on memory tasks varied considerably among individual aged subjects (Bartus R.T. et al., 1982, *Science,* 217:408-417).

Neurological diseases provide a unique series of complications for clinicians, patients, and care givers; the diseases often progress rapidly and disrupt a vast number of major life functions. The progressive nature of these diseases makes the passage of time a crucial issue in the treatment process.

EP0804615 (WO 95/29257) provides insight into the treatment of neurodegenerative diseases, such as Alzheimer's disease, using cholinomimetic therapy. EP08046I5 describes methods for determining the clinical responsiveness of a subject with Alzheimer's disease to a cholinomimetic therapy by determining the subject's apolipoprotein E2, E3, and E4 allele load, wherein the presence of apoE2 or E3 in a biological sample from the subject indicates a predisposition to respond to a cholinomimetic-based therapy, and the presence of apoE4 indicates a predisposition not to respond to a chotinomimetic-based therapy. EP0804615 does not correlate apoE allele load to a prognosis protocol, which includes, for example, relative age of onset and rate of progression, nor does it describe the use of apoE allele determination for proposing the clinical responsiveness of a subject having a neurodegenerative disease other than Alzheimer's disease to a choliriomimetic therapy.

WO 96/02670 describes a method of prognosing the disease progression and ultimate degree of dementia that a subject would suffer during a beta amyloid-related disease by detecting the absence or presence of ApoE alleles or isoforms, wherein the presence of an ApoE2 isoform or allele in a subject at risk of, or in the early stages of, such a disease, indicates a milder course of the disease. WO 96/02670 is solely directed to determining whether a subject would have a severe or benign course of a beta amyloid-related disease; it provides no description as to prediction of drug efficacy in these subjects, nor does it suggest the actual steps of a prognosis protocol.

WO 96/03656 describes a method of prognosing the likelihood of the development of chronic neurodegenerative pathology in a head-injured subject, or a subject at risk of head injury, by detecting ApoE isoforms in the subject. WO 96/03656 does not disclose that apoE isoform detection can be used to predict drug efficacy in a head-injured subject, or a subject at risk of head injury, and does not discuss the actual steps of a prognosis protocol.

Treatment choices for neurological disease are frequently complicated by the fact that it often takes a significant period of treatment to determine whether or not a drug is having a therapeutic effect Accordingly, treatment with the most effective drug or drugs is often delayed while the disease continues to progress. A method which would allow one to predict which patients will respond to specific therapeutics and dosages would provide physical and psychological benefits. As healthcare becomes increasingly inaccessible, the ability to allocate healthcare resources effectively also becomes increasingly important.

### SUMMARY OF THE INVENTION

We have discovered a method for predicting the prognosis for patients having a neurological system disease. Specifically, we have found a method for determining responsiveness to cholinornimetic drug therapies, the relative age of onset, and the relative rate of progression of various diseases. From these discoveries we have developed an approach, referred to herein as phatmacogenetics, for profiling patients already diagnosed with a neurological disease. Pharmacogenetics provides a prognosis for the patient, including determination of the most effective drug (where appropriate) and drug dosage (if any). Our method allows clinicians, patients, and family members to make informed choices about therapeutic regimes. Furthermore, where more than one therapeutic exists, the pharmacogenetic methods of the invention allow the clinician to avoid serially administering therapeutics until a therapeutic which works is discovered through trial and error, with pharmacogenetics an appropriate therapeutic can be administered on the first round of therapy. This method will provide more rapid treatment with the appropriate drug and drug dose. Even where drug therapy is inappropriate the method will provide patients with a prognosis and will allow the patient the option of avoiding what may be needless drug side-effects.

The pharmacogenetic method has an additional advantage when utilized in the field of drug testing. The method allows the patient being considered for enrollment in a drug trial to be classified as likely or unlikely to benefit from a cholinomimetic therapy. If the patient is likely to respond to a cholinomimetic, and the drug being tested is not a cholinomimetic, then the patient may be physically unsuitable for the trial or it may be ethically inappropriate. Conversely, patients likely to respond to cholinomimetics will provide more statistically useful data on novel cholinomimetic therapeutics. Patients unlikely to respond to cholinomimetic drugs are particularly good candidates for non-cholinomimetic therapies from both a physical and ethical perspective.

In one aspect, the invention provides a method of creating a prognosis protocol for a patient diagnosed with neurofibromatosis, a pathology of the developing nervous system, depression, a nervous system injury, an infection of the nervous system, coma, multi-infarct dementia, a dietary deficiency, or a cardiovascular injury, said method comprising:
a) identifying a patient already diagnosed with said disease;
b) determining the *apoE* allele load of said patient by genotyping or phenotyping, said phenotyping including characterizing an ApoE protein isoform; and
c) converting the data obtained from step b) into a prognosis protocol, said prognosis protocol including a prediction of drug efficacy and patient outcome, wherein the presence of one or more apoE4 alleles in said patient is indicative of said patient being unlikely to benefit from cholinomimetic therapy.

The prognosis protocol may include a prediction of drug efficacy, a prediction of patient outcome, or both. In preferred embodiments of these aspects, the methods may further include the steps of obtaining a patient profile, which may, preferably, include the patient's sex and/or genotype (e.g., presenilin, apolipoprotein E, or genotype).

For example, the patient may have been diagnosed with a congenital defect in amino acid metabolism (e.g., argininosuccinicaciduria, cystathioninuria, histidinemia, homocystinuria, hyperammonemia, phenylketonuria, and tyrosinemia); fragile X syndrome; stroke; or multi-infarct dementia.

In a further aspect, the invention provides a method for identifying patents suitable for participation in a clinical trial of a drug for the treatment of a disease selected from the group consisting of neurofibromatosis, a pathology of the developing brain, depression, amyotrophic lateral sclerosis, multiple sclerosis, a nervous system injury, an infection of the nervous system, a prion disease, coma, multi-infarct dementia, a dietary deficiency, or a cardiovascular injury, said method comprising:
a) identifying a patient with said disease;
b) determining the *apoE* allele load of said patient by genotyping or phenotyping, said phenotyping including characterizing an ApoE protein isoform; and
c) converting the data obtained from step b) into a prognosis protocol, said prognosis protocol indicating whether or not said patient is a candidate for a cholinomimetic drug trial or non-cholinomimetic drug trial, wherein a patient lacking both apoE4 alleles is expected to be a candidate in a cholinomimetic drug trial or wherein a patient having one or more apoE4 alleles is expected to be a poor candidate in a cholinomimetic drug trial.
prognosis protocol provides an indication of whether or not said patient is a candidate for a cholinomimetic drug trial or non-cholinomimetic drug trial. Patients who have one or more apoE4 alleles are poor candidates for the trial of a cholinomimetic drug trials.

The present invention also provides a method for determining the risk of stroke in a patient, said method comprising:
a) determining the *apoE* allele load of said patient by genotyping or phenotyping, said phenotyping including characterizing an ApoE protein isoform; and
b) determining the BChE genotype of said patient, wherein the presence of one or more apoE4 alleles and one or more BChE-K alleles in said patient indicates said patient is at risk of stroke.

The invention further provides a method for performing pharmacogenetic analysis, said method including a means for converting a patient profile into a prognosis protocol, wherein said means includes determining the *apoE* allele load of a patient diagnosed with neurofibromatosis, a pathology of the developing nervous system, depression, a nervous system injury, an infection of the nervous system, coma, multi-infarct dementia, a dietary deficiency, a cardiovascular injury, or stroke by genotyping or phenotyping, said phenotyping including characterizing an ApoE protein isoform.

Therapeutic agents relevant to the present invention include both cholinomimetic and non-cholinomimetic drugs used for the treatment of neurological disease. Cholinomimetic drugs may be selected from the group consisting of inhibitors of acetylcholine degradation, inducers of acetylcholine synthesis, acetylcholine agonists or mimics, and musearimc M2-receptor antagonists. It should be noted that the therapies suggested by the pharmacogenetic method may be used alone, or in combination with other known therapies that are not otherwise contraindicated for the patient.

For the purpose of the present invention the following terms are defined below.

"Cognitive enhancers" means drugs which enhance a) memory performance, whether it is verbal memory, spatial memory, or factual memory and b) learning capacity.

"Cholinomimetic therapies" means drugs that mimic the function of acetylcholine or enhance the activity of remaining acetylcholine synthesizing cells. These drugs include, but are not limited to, inhibitors of acetylcholine degradation (acetylcholine esterase inhibitors like tacrine), drugs that mimic acetylcholine structure and function (agonist: muscarinic M1-receptor agonist is a typical example), drugs that block acetylcholine uptake by neurons and drugs that interact pre-synaptic receptors to induce acetylcholine release from cholinergic neurons.

"apoE probes" means nucleic acid probes or apoE allele specific antibodies that selectively recognize the apoE2, apoE3 or apoE4 alleles or proteins, respectively.

"Non-AD neurological disease" means any disease other than Alzheimer's. disease, which involves the neuronal cells of the nervous system. Specifically included are: prion diseases (e.g, Creutzfeldt-Jakob disease); pathologies of the developing brain (e.g., congenital defects in amino acid metabolism, such as argininosuccinicaciduria, cystathioninuria, histidinemia, homocystinuria, hyperammonemia, phenylketonuria, and tyrosinemia, and fragile X syndrome); pathologies of the mature brain (e.g., neurofibromatosis, Huntington's disease, depression, amyotrophic lateral sclerosis, multiple sclerosis, and stroke); conditions that strike in adulthood (e.g. Creutzfeldt-Jakob, disease, Huntington's disease, Lewy body disease, Parkinson's disease, Pick's disease, amyotrophic lateral sclerosis, multiple sclerosis, neurofibromatosis), brain injury, stroke, and, multi-infarct dementia; and pathologies of the brain (e.g., brain mishaps, brain injury, coma, infections by various agents, dietary deficiencies, and cardiovascular accidents).

"Alzheimer's Disease (AD)" means a pathology characterized by an early and extensive loss of entorhinal cortex neurons. AD patients may be identified by progressive and degenerative effects on the brain which are not attributable to other causes. Post-mortem, the disease may be diagnosed by the presence of plaques and fibrils.

"Already diagnosed" means already diagnosed as having the neurological disease, having a genetic predisposition to the disease, or both.

''Patient profile" means data pertaining to the patient for whom the pharmacogenetic analysis is being performed. Data may include information on the patient's diagnosis, age, sex, and genotype. The patient's profile may also include materials from the patient such as blood or purified RNA or DNA.

"ApoE genotyping" means determination of the type and number of apoE alleles present in the patient, whether determined by nucleic acid sequencing, PCR or RT-PCR amplification, examination of apoE protein, or by other methods available to those skilled in the art.

"Allele load" means the relative ratio of apoE2, 3, and 4 alleles in the patient's chromosomal DNA. The allele load may be determined by comparing the relative numbers of the patient's already known apoE allele types.

"PCR or RT-PCR amplification" means subjecting a DNA sample to a Polymerase Chain Reaction step or an RNA sample to a Reverse Transcriptase-Polymerase Chain Reaction step, such that, in the presence of appropriately designed primers, a DNA fragment is synthesized or fails to be synthesized and thereby reveals the allele status of a patient.

"BCHE-K allele" means the polymorphism of the butyrylcholinesterase (BChE) gene which has a point mutation at nucleotide 1615 that changes amino acid residue 539 from alanine to threonine and can result in an enzyme with reduced catalytic activity other mutations. Other polymorphisms of this locus exist (e.g., deletions (BCHE*FS4), missense mutations (BCHE*24 M, *1005, *250P, *267R, *3301, *365R, *418S, *515C, *539T), and nonsense mutations (BCHE*119STOP, *465STOP)) and are included within the scope of the invention.

"Prognosis protocol" means a therapy plan provided to the clinician or patient using the pharmacogenetic method. The prognosis protocol includes an indication of whether or not the patient is likely to respond positively to a cholinomimetic therapeutic. In preferred embodiments, the protocol also includes an indication of the drug dose to which the patient is most likely to respond.

"The pharmacogenetic method" is a method whereby genetic and diagnostic data, including the patient's neurological diagnosis and the patient's apoE and/or BChE genotype are processed to provide therapeutic options and prognoses.

### BRIEF DESCRIPTION OF THE DRAWINGS

Fig. 1 is a Western blot analysis of the apoE protein levels in the hippocampus of AD and non-AD individuals.
Fig. 2 is a Northern blot analysis of apoE mRNA expression in the hippocampus of AD and non-AD individuals.
Figs. 3A-3F are graphs illustrating E4 allele copy number, tangles and senile plaque densities in the hippocampus in AD.
Figs. 4A and 4B are graphs illustrating E4 allele copy number and choline acetyltransferase activity in Alzheimer's disease.
Figs. 5A and 5B are graphs illustrating the loss of neurons which synthesize acetylcholine in the nucleus basalis of Meynert and in the diagonal band of Broca.
Figs. 6A-6J are graphs illustrating the effect of apo E4 allele copy number on a) choline acetyltransferase activity, b) nicotinic receptor density, c) total muscarinic receptor density, d) muscarinic M1 (post-synaptic) and e) muscarinic M2 receptor density in post-mortem non-AD and AD brains in the hippocampal formation and temporal cortex.
Fig. 7 is a graph illustrating the individual neuropathological and morphological characteristics associated to the cholinergic system in the brain of the various Alzheimer's disease and control cases investigated.
Figs. 8a and 8b are graphs illustrating the AD assessment scale (ADAS) delta values (end values minus screen values) in tacrine-treated AD patients with different apoE genotype.
Figs. 9A and 9B are graphs of the ADAS-COG Scores with tacrine and xanomeline, respectively, from day 0 to week 30.
Fig. 10 is a graph of the effect of the presence of the apoE4 allele on the ADAS-COG scores in Figs. 9A and 9B.
Fig. 11 is a graph of the brain apoE levels in AD subjects as a function of the apoE allele load.

### DETAILED DESCRIPTION OF THE INVENTION

Here we show that correlation of age, sex, genotype, apoE allele load, and presence of the BChE-K allele, may be used to determine the appropriate drug or drugs, dosage, and prognosis for a given AD or non-AD patient. The prognosis can include a prediction of both relative age of onset and rate of progression.

Losses of cholinergic neurons and/or choline acetyltransferase (ChAT) activity are well known hallmarks of AD (Perry E.K. et al, 1977, *J. Neurol. Sci.,* 34:247 265; Davies P. et al, 1976, *Lancet,* 2:1403). We have investigated the relationship between the apoE4 genotype and cholinergic deficits, and we observed that the greater the number of apoE4 alleles the lower the apoE level. Furthermore, reduction in ChAT activity in the hippocampus and temporal cortex of AD cases is inversely proportional to the apoE4 allele copy number (i.e. where the apoE4 allele copy number is increased the ChAT activity is decreased). In addition, we found that another presynaptic marker of cholinergic projection, the nicotinic receptor, was markedly reduced in apoE4 AD subjects. Conversely, we have found that a typical post-synaptic marker, M1-muscarinic receptor, is unaltered in AD versus non-AD subjects, irrespective of whether apoE4 is present or not. The M2-muscarinic receptor, a composite pre- and post-synaptic marker, is also unaffected by the apoE4 allele gene dosage. We have also observed that the presence of the apoE4 allele lowers the age of onset of neurological disease and worsens the prognosis.

The above findings clearly indicate the existence of distinct genetic entities in neurological disease which correlate with differential degrees of alterations of cholinergic innervation. In turn, the innervation level correlates with the prognosis, including the ability to respond to cholinomimetic drugs.

We believe the correlation between apoE4 allele load and reductions in ChAT activity and nicotinic receptors may be explained by at least two distinct phenomena. First, phospholipids such phosphatidylcholine (PC) and phosphatidylethanolamine (PE), that can serve as precursors to choline in the synthesis of acetylcholine (Ach), could be transported into neurons via the classical apoE-LDL receptor pathway. An isoform-dependent impaired regulation of the transport of phospholipids in the brain of apoE4 carriers could explain the reduced levels of PC, PE and choline reported in AD (Pettegrew J.W., 1989, *Ann. NY Acad Sci.,* 568:5-28; Nitch RM et al., 1992, *Proc. Natl. Acad. Sci.,* 89:1671-1675). This, in turn, may lead to decreased Ach synthetic capacities. This hypothesis is consistent with membrane defects reported in AD subjects such as changes in membrane fluidity in the hippocampus and in the platelets of AD patients. The loss of cholesterol reported in AD and the effect of apoE4 on nicotinic binding activity are consistent with the apoE4/impaired lipid homeostasis hypothesis.

In addition to the above, the reduction in neuronal ChAT activities and choline levels in both AD and non-AD patients could parallel the loss of cholinergic neurons. The analysis of the number of acetylcholinesterase-positive neurons in the nucleus basalis of Meynert (NBM) and the diagonal band of Broca (DBB) in AD patients revealed marked losses of cholinergic neurons in apoE4 carriers versus apoE3 homozygous AD cases.

Although initially made in AD and stroke patients, we believe our observation regarding apoE allele load and drug therapies can be generalized to non-AD neurological diseases because the underlying mechanism altered by the apoE allele load is not AD-specific. Our discovery indicates that the apoE4 allele load, taken together with the patient profile parameters, can predict individual variations in brain cholinergic systems. Other useful predictions pertain to the appropriate therapies for diseases such, Parkinson's disease, and multiple sclerosis in view of the apoE4 allele load and BChE allele status. Prospective-retrospective analyses of patients which are either good or poor responders to cholinomimetics as well as to other drugs designed to ameliorate the conditions of stroke, Parkinson's disease, and multiple sclerosis, (e.g., aspirin, antithrombotics, ticlopidine HCl (Ticlid™), levodopa-carbidopa, (Sinemet™) and interferon β-1B (Betaseron™)) are presented in the examples.

Cholinomimetic drugs are known to enhance cognitive performance in both young and old subjects carrying at least one apoE2 or apoE3 allele. In apoE2 and apoE3 individuals cognitive performance could be restored or enhanced by the administration of cholinomimetics such as acetylcholine agonists (e.g. the M1-agonist xanomeline, available from Eli Lilly), M2 receptors-antagonist (e.g., BIBN-99 from Boehringer Ingeilheim), inhibitors of acetylcholine degradation (e.g., tacrine, available from Parke-Davis, or E-2020, available from Pfizer) in subjects carrying apoE2 or apoE3 but not apoE4. Pharmacogenetics allows the physician to select the most appropriate treatment from the several available, for a given patient.

The present invention will be more readily understood by referring to the following examples which are given to illustrate the invention rather than to limit its scope.

### EXAMPLE I

### Determination of apoE levels and allele load

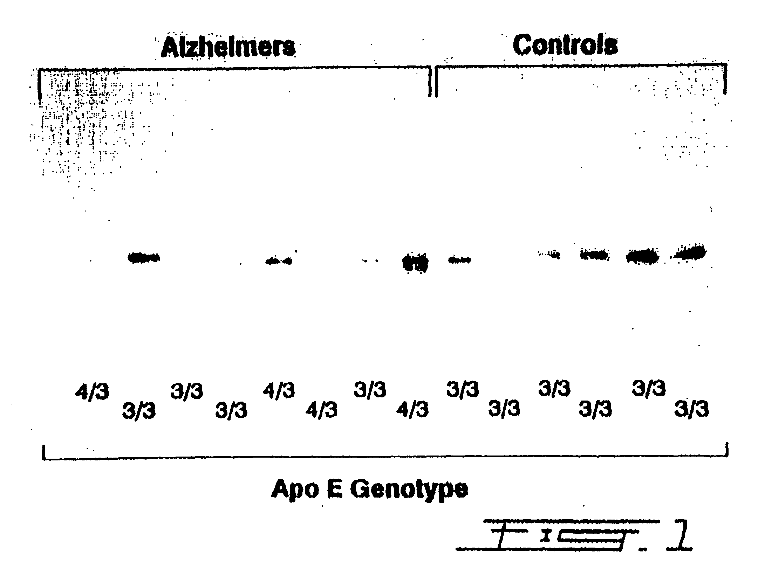

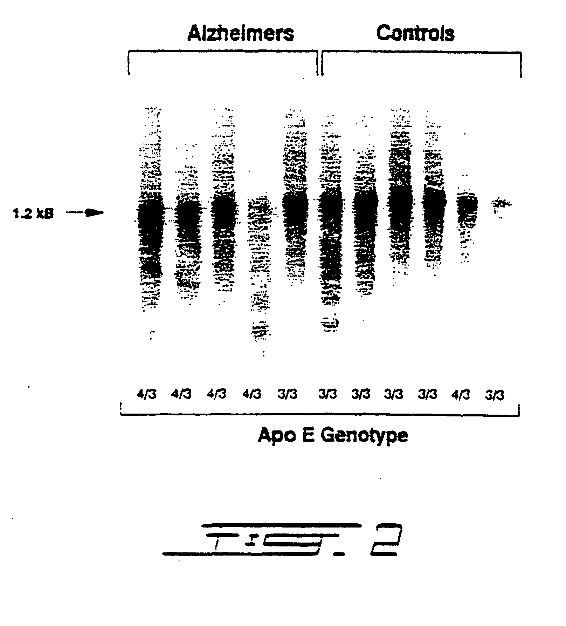

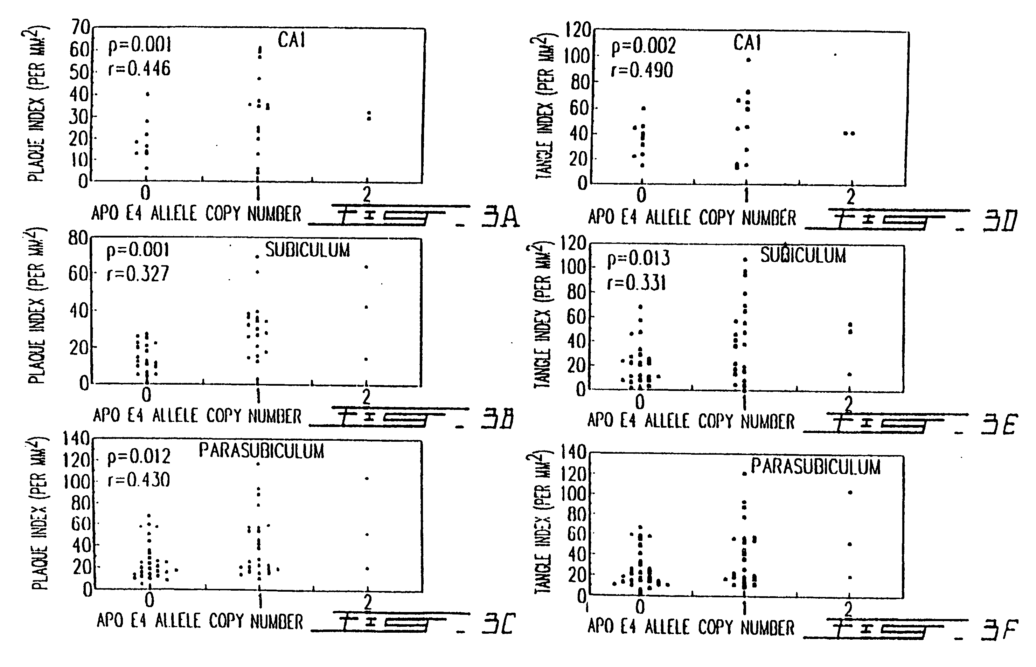

Figures 1 and 2 illustrate Western blot and Northern analyses of apoE levels in the hippocampus of non-AD and AD patients as a function of their respective genotype.

Frozen hippocampi from post-mortem patients were obtained from the Douglas Hospital Brain Bank in Montréal. Age and sex were matched and post-mortem delays were similar in both groups (~14 hrs). Post-mortem delays up until 24 hours had little impact on apoE stability (Lehtimaki T., 1991, *Clin. Chim. Acta,* 203:177-182) and apoE-containing tissue can be stored at -80°C for several months without noticeable degradation. Hippocampal total RNA was extracted and quantified by oligo (dT) hybridization as previously described (Poirier J. et al., 1991, *Mol. Brain. Res.,* 11:97-106). The hybridization protocol using the full length apoE cRNA probe used in this experiment has also been previously described (Poirier J. et al., 1991, *Mol. Brain. Res.,* 9:191-195). High molecular weight DNA was isolated from frozen cerebellum or temporal cortex tissue as adapted from Goelz et al. (Goelz S.E. et al., 1986, *Biochem. Biophys. Res. Comm.,* 130:118-126).

The apoE genotype was determined by an allele-specific primer extension of purified brain DNA using a modification of the method of Main et al. (Main R.F. et al., 1991, *J. Lipid. Res.,* 32:183-187). The primers labeled D, E, F, G, and H were synthesized for us by Genosys Biotech (The Woodland, TX) using primer sequences provided in Main et al. (Main R.F. et al., 1991, *J. Lipid. Res.,* 32:183-187). Reactions were carried out in a volume of 50uL containing 1 ug of DNA; deoxyadenosine triphosphate, deoxycytidine triphosphate, deoxythymidine triphosphate and deoxyguanosine triphosphate, each 0.2 mmol/L; 10% dimethyl sulfoxide; 12.5 pmol of either primer D, E, F, or G; 25 pmol of primer H; and 10 uL of 10x PCR reaction buffer (Vector Biosystem, Toronto, ONT.). The DNA in the reaction mixture was first denatured for 10 min. at 96°C and then cooled to 4°C. One unit of Taq polymerase (Vector Biosystem, Toronto, ONT.) was then added to each sample. Each sample was reheated for 2 min. at 96°C and subjected to 30 cycles in a thermal cycler with each cycle consisting of a 10 sec denaturation at 96°C, 30 sec annealing at 58°C, and 1 min. extension at 65°C. The reaction products were visualized by electrophoresis of 10uL of the reaction mixture in a 1% agarose gel containing TPE buffer (0.08 mol/L Tris-phosphate, 0.002 mol/L EDTA, Sigma, St-Louis, USA) and ethidium bromide (0.15 ug/mL) for 1 hr at 67v. The gels were then photographed and the banding profile was compared to known standards.

Analysis of apoE protein was performed by immunoblot. Briefly, 50 ug of hippocampal homogenate, pre-treated with 1 u of neuraminidase, was loaded on a 25 cm long SDS polyacrylamide gel (10%) and run for 3 hours at room temperature. Proteins were transferred onto a nitrocellulose filter using a BIORAD™ Trans-blot cell and detection of the apoE protein was performed using a polyclonal antibody raised against human apoE protein (International Immunology Corp., CA, Dil. 1:2000). Adsorption of the antibody with purified apoE completely blocked the detection of the human apoE protein which has a MW of 34-36 kDa. Molecular weight markers (Rainbow markers, Amersham) were run in adjacent wells while visualization of the bands was done with a chemiluminescence detection kit (Amersham, Cat. No. RPN 2100). Quantification of the autoradiographic signals was performed using a MCID image analysis system (Ste-Catherine, Ontario) equipped with the ID-gel analysis software.

### Results

In the AD brain, apoE mRNA was shown to be present but not up-regulated in response to cell loss and deafferentation. We have proposed that the synaptic loss reported in apoE4/AD subjects could be the result of a selective impairment of the apoE/LDL receptor pathway. Figure 1 illustrates levels of apoE measured in the brain of AD subjects with different genotypes as well as in control subjects with apoE3/2 and apoE3/3 genotypes. Of the ~90 neuropathological elderly control subjects that we have examined so far (using standard criteria), none of those fitting the AD criteria were found to carry the apoE4 allele. A significant reduction in apoE concentration was measured in the tissue of apoE4 carriers versus non-apoE4 subjects. Interestingly, the risk of developing AD, the accumulation of senile plaques and tangles in the AD brain, and the loss of cholinergic function in AD follow a genotype gradient: increase in damage from E2/2 ---> E3/3 ---> E4/4, the latter genotype representing the worse case scenario.

We believe that the most important observation from this data is the fact that the AD pathology is much more severe (cell loss, deafferentation, and increased GFAP expression, etc.) in apoE4 AD carriers than it is in the apoE4-negative AD subjects. Yet, apoE levels, which should be increased in response to damage and cell loss, are in fact decreased in those same apoE4 individuals.

### Pathophysiological consequences of having low apoE levels if you are a life long apoE4/AD carrier

In order to address the consequences of being an apoE A4 carrier, we have examined apoE/apoB (LDL) receptor expression (cholesterol internalization) and HMG-CoA reductase expression (cholesterol synthesis) in the hippocampus of AD and control subjects with different apoE genotypes to assess the consequences of the poor delivery of lipids in apoE4 carriers. Table 1 summarizes the mRNA prevalences measured for the LDL receptor, HMG-CoA reductase, and glial fibrillary acidic protein (GFAP) in post-mortem brains of control and AD subjects with known apoE genotypes. Beta-tubulin was used as a non-changer transcript to adjust for RNA loading in the gels. GFAP mRNA prevalence, a well known marker of local tissue damage, was markedly increased in apoE4 carriers when compared to apoE3/3 AD subjects: supporting the notion that more cellular damage was present in the brain tissue of apoE4 carriers. The LDL receptor and HMG-CoA reductase mRNA prevalence were found to be increased in AD subjects carrying the apoE4 allele when compared to non-E4 carriers. This double induction of cholesterol internalization and synthesis was consistent with results obtained in fibroblasts and macrophages cultured in the absence of cholesterol in which, both cholesterol uptake (via the LDL receptor) and synthesis (via the HMG-CoA reductase) were markedly up-regulated to facilitate a rise in intracellular cholesterol.

**Table 1.**

| mRNA Prevalence in the Hippocampus of Brain Obtained from AD Subjects | | | |
|---|---|---|---|
| | Apo E3/3 | Apo E4/3 | Apo E4/4 |
| Genes | % OF CONTROLS | | |
| GFAP | 110 | 365* | 345* |
| LDL | 86.5 | 173* | 170* |
| HMG-CoA | 87 | 191* | 152 |
| | N=6 | N=7 | N=3 |

| | | | |
|---|---|---|---|
| *: p<0.05 | | | |

In other words, the reduction of brain apoE in apoE4 carriers (the only lipoprotein carrier of the CNS) although moderate in size, was sufficient enough to shift cholesterol metabolism from its normal steady state concentration to a depleted state. This forces cells to compensate by up-regulating both synthesis and internalization. It is thus quite conceivable that apoE depletion observed in the brain of apoE4 carriers may significantly compromise lipid delivery in the CNS.
These results provide an intriguing new explanation for the reported reduction of cholesterol in the brain of AD subjects with unknown genotype.

### EXAMPLE II

### Correlations between apoE4 allele copy number and senile plaques and neurofibrillary tangles in three different areas of the hippocamnus. namely the CA 1 sub-field. subiculum and the parasubiculum in individuals with different apoE4 allele copy number.

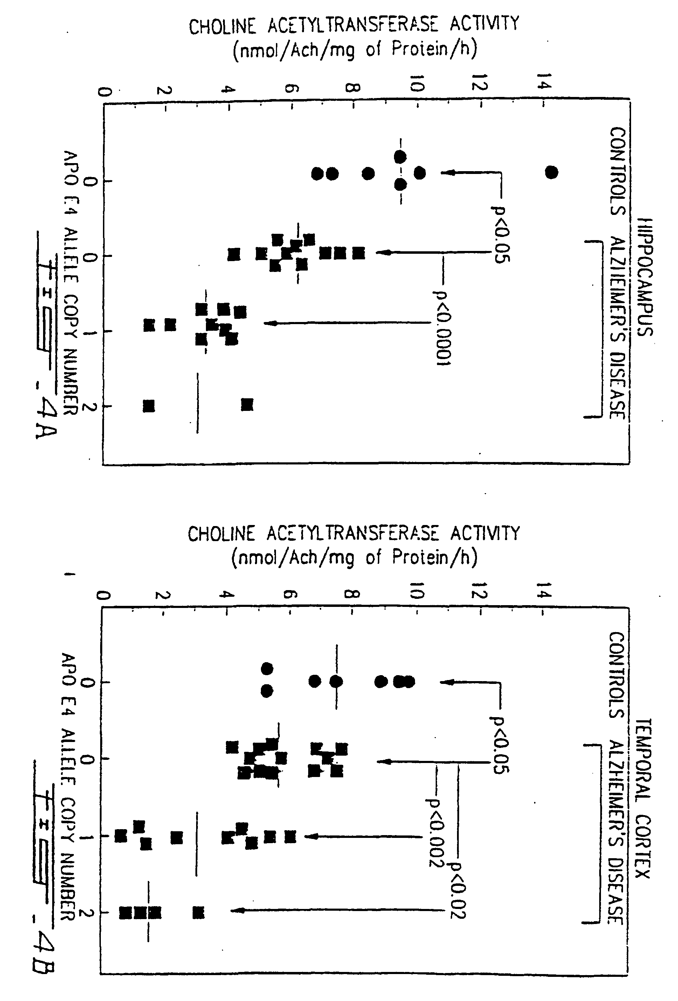

The results are summarized in Fig. 3 and below.

### Methods

Genotype was determined as described for Fig. 1. Senile plaque and tangle density measurements were performed as described before (Aubert I. et al., 1992, J. *Neurochem.,* 58:529-541). Paraffin embedded hippocampal tissue from 59 autopsied AD patients was obtained from the Douglas Hospital Brain Bank and stained with hematoxylin and eosin, modified Bielchowsky, and alkaline Congo™ red. Quantitative morphometric evaluations of neurofibrillary tangles and senile plaques were performed as follows. A micrometric scale was used for calibration. Readings were done with a 10 X objective for plaques and a 25X objective for tangles. Diffuse plaques were excluded from these measurements. Screening of alkaline Congo™ red stains under polarized light was used to control the reliability of tangle staining and, to a lesser extent, of senile plaque's affinity for the modified Bielchowsky stain. Idiopathic Parkinson's disease (IPD) was diagnosed according to the presence of significant loss of pigmented neurons, presence of Lewy bodies in residual neurons, clusters of macrophages, and gliosis in the pars compacta of the substantia nigra. These histological features usually correlate with pre-mortem classical features of IPD, such as resting tremor, rigidity, and akinesia. Statistical analysis was performed using the Multivariate General Linear™ Models as part of the Systat Statistical Software™ package.

### Results

The correlation between apoE4 allele copy number and the density of senile plaque is very strong in all three hippocampal regions. The correlation between apoE4 allele copy number and the neurofibrillary tangles index was also significant for the CA1 and the subiculum areas. These results support the concept that apoE4 plays a role in the pathophysiology of AD.

### EXAMPLE III

### Association between neurological pathology and acetylcholine activity.

Brain phospholipids such as phosphatidylcholine (PC) and phosphatidylethanolamine (PE) have been shown to play an important role in the availability of choline, the rate-limiting precursor of acetylcholine (Ach). Brain levels of choline are decreased by 40-50% in AD frontal and parietal cortex (Nitch RM et al., 1992, *Proc. Natl. Acad. Sci.,* 89:1671-1675). Similarly, cholesterol is apparently required for the proper function of some cholinergic receptor sub-types (Jones O.T. & McNamee M.G., 1988, *Biochemistry,* 27:2364-2374). On the basis of possible interrelationships between apoE4, senile plaque, and neurofibrillary tangle counts and Ach, we evaluated the association between the presence of apoE4 and cholinergic dysfunction, a classical hall mark of AD (Bowen DM et al., 1981, *N. Engl. J. Med.,* 305:1016; Whitehouse PJ et al., 1982, *Science,* 215:1237). We focused our attention on the determination of ChAT activity, the key enzyme involved in the synthesis of Ach, in post-mortem hippocampus and temporal cortex of individuals suffering from AD and in control subjects.

### Methods

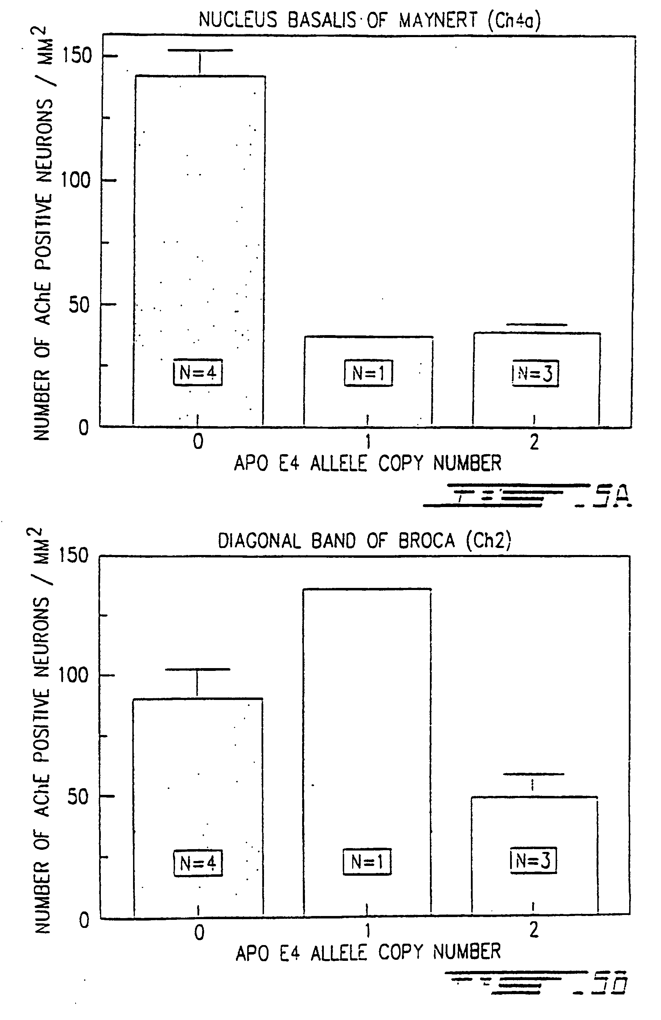

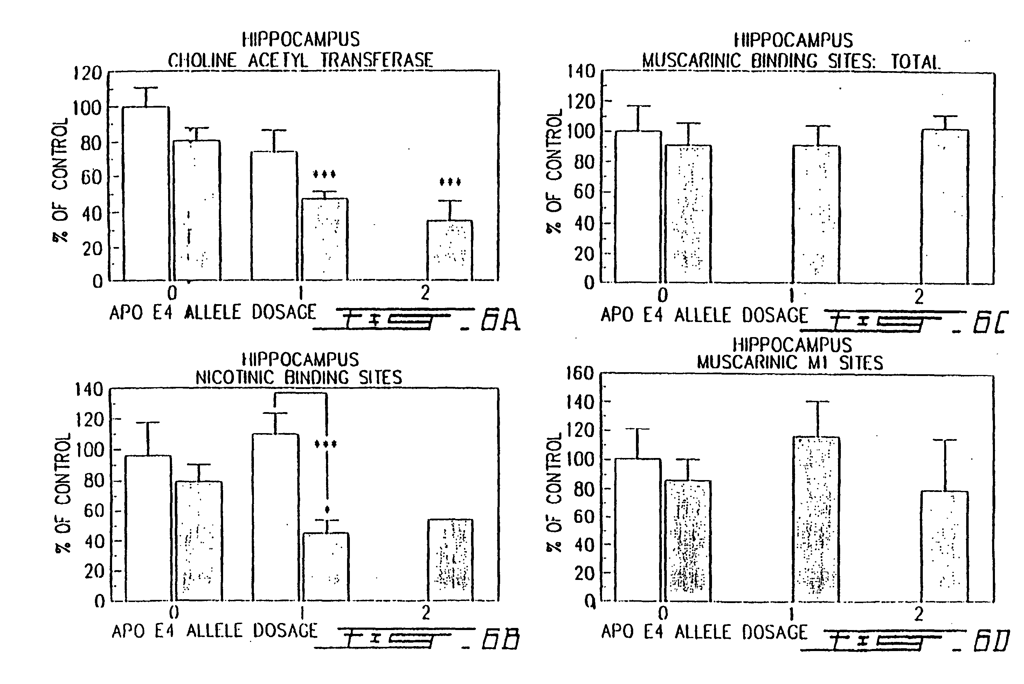

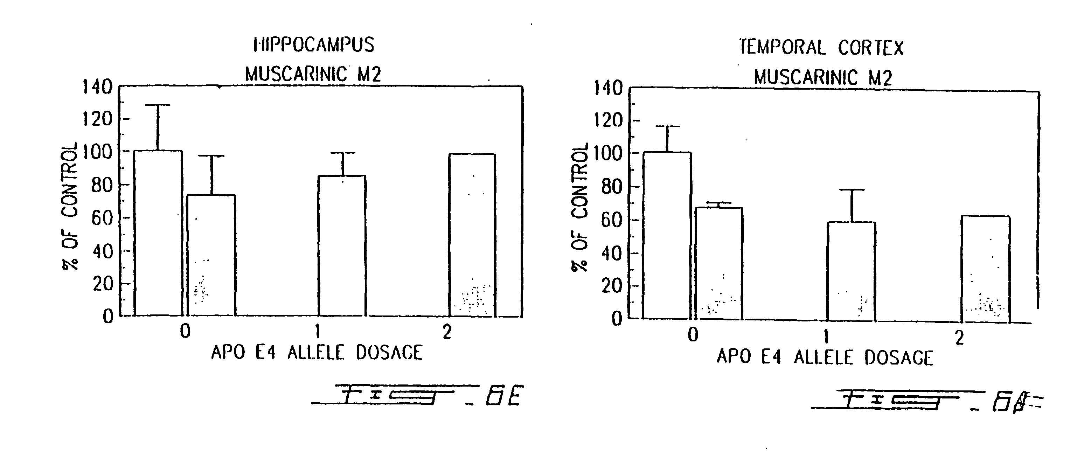

Figs. 4 and 6 illustrate the effect of the presence of apoE4 isoform on hippocampal and temporal cortex ChAT activity.

Left hemisphere brain tissue, for biochemical assays, was sectioned into thick (10 mm) coronal slices and quickly frozen in 2-methylbutane at -40°C before storage at -80°C. Tissues from hippocampal and temporal cortical areas were homogenized and incubated for 15 min. in buffer containing [14C] acetyl-CoA as previously described in detail elsewhere (Aubert I. et al., 1992, *J. Neurochem.,* 58:529-541). The post-mortem diagnostic was performed as described for Fig. 3. Apolipoprotein E genotype was determined as described for Fig. 1.

### Results

Highly significant reductions in ChAT activity were seen in apoE4 carriers. In the hippocampus (22 ADs and 7 controls), ChAT activity values are 9.44± 0.93 (control apo E3/3, no apoE4 allele), 6.09± 0.36 (AD apoE3/3, no apoE4 allele), 3.21± 0.31 (AD apoE4/3) and 2.94± 1.52 (AD apoE4/4) nmol Ach/mg protein/hr, respectively. Statistical analyses indicate that control apoE3/3 ChAT values were significantly different from the AD apoE3/3 group (p<0.05) and the apoE4/3 group (p<0.0001) whereas, AD apoE3/3 ChAT levels were significantly different from the AD apoE4/3 group (p<0.0001) and likely the apoE4/4 group, although statistical evaluation could not be performed because of the limited number of apoE4 homozygotes available. Similar results were obtained in the temporal cortex (26 AD subjects and 7 controls) with ChAT activity values of 7.48± 0.74 (control apoE3/3), 5.65± 0.33 (AD apoE3/3), 2.91 ± 0.66 (AD apoE4/3) and 1.56± 0.47 (AD apoE4/4) nmol ACh/mg protein/hr, respectively. Statistical analyses indicate that control apoE3/3 ChAT values were significantly different from all AD groups (p<0.05 for apoE3/3 and p<0.001 apoE4/3) whereas AD apoE3/3 ChAT levels were significantly different from the AD apoE4/3 group (p<0.002) and the apoE4/4 group (p<0.02).

Without wishing to find ourselves bound to a particular explanation, we propose the apoE4 allele copy number-rated reduction in ChAT activity and nicotinic receptors may be caused by at least one of two distinct phenomena. First, phospholipids such as PC and PE which can serve as precursors to choline in the synthesis of Ach could be transported into neurons via the classical apoE-LDL receptor pathway. An isoform-dependent impaired regulation of the transport of phospholipids in the brain of apoE4 carriers could explain the reduced levels of PC, PE, and choline reported in AD; this then leading to decreased Ach synthetic capacities. Alternatively, the reduction in neuronal ChAT activities and choline levels could be secondary to losses of cholinergic neurons. Analysis of the number of acetyl cholinesterase-positive neurons in the nucleus basalis of Meynert (NBM) and the diagonal band of Broca (DBB) of a small number of AD patients (n=7) revealed marked losses of cholinergic neurons in apoE4 versus apoE3 homozygous AD cases (a 70% decrease in the NBM and a 45% decrease in the DBB, Fig. 5).

These results clearly indicate that there are distinct genetic components to neurological disease and that diseases such as AD, for example, show different degrees of altered cholinergic activity, as revealed by ChAT activity and presence of nicotinic receptors. Our data also suggest that cholinergic function in apoE3/3 carriers may be spared and that these patients could be better responders to a cholinomimetic-based therapy.

### EXAMPLE IV

### Additional Neurochemical Alterations and Therapeutic Response in Subjects Treated with Cholinomimetics

### Methods

### i) Post-Mortem Study of Cholinergic Marker

Case Selection and apoE genotyping: Frozen tissues from 84 autopsy confirmed cases of sporadic AD (35 females, 77.3+ 8.7 years: 49 males, 76.1 + 9.5 years) and 14 control individuals (8 females, 71.6+3.6 years; 6 males, 66.4+3.1 years) were obtained from the Douglas Hospital Brain Bank in Montréal, Canada. The average post-mortem delay was 17.2 + 1.3 and 20.0 + 4.6 hours for AD and control subjects, respectively. It should be noted that the availability of apoE4/4 homozygous subjects is very limited, probably due to the fact that apoE4/4 homozygous individuals represent less than 1% of the entire population.

High molecular weight DNA for genotype analysis was isolated from frozen cerebellum or temporal cortex tissue as adapted from (Nalbantoglu J. et al., 1994, *Ann. Neurol.* 36:889-895). The apoE genotype was determined by allele-specific primer extension of purified brain DNA using primers and methods as described in Example I.

Neuropathological Analyses: Neurofibrillary tangle and senile plaque indices were determined as described in detail elsewhere (Aubert I. et al., 1992, *J. Neuro chem.,* 58:529-541; Etienne P. et al, 1986; *Neuroscience,* 19:1279 1291). Hippocampal tissue sections (15 µm; paraffin embedded) were stained with either hematoxylin and eosin, modified Bielchowsky stain, or alkaline Congo™ red to visualize neurofibrillary tangles and senile plaques. Quantitative morphometric evaluation of neurofibrillary tangles and senile plaques was performed using a micrometric scale for calibrated readings using a 10X objective for senile plaques and a 25X objective for neurofibrillary tangles. Diffuse plaques were excluded from all measurements. Screening of alkaline Congo™ red stains under polarized light was used to insure the reliability of neurofibrillary tangle staining and, to a lesser extent, senile plaques' affinity for modified Bielchowsky stain. These criteria are consistent with those used in the classification of Khachaturian (Aubert I. et al., 1992, *J*. *Neurochem.,* 58:529-541).

### ii) Cholinergic Function in the Post-mortem Brain of Control and AD Subiects

Materials: [3H]QNB (45.7 Ci/mmol), [3H]PZ (87.0 Ci/mmol), [3H]AF-DX 116 (49.3, 57.0, or 70.0 Ci/mmol), [3H]MCC (84.5 Ci/mmol), and [4C]acetyl-CoA (48.8 mCi/mmol) were purchased from New England Nuclear (Boston, MA, U.S.A.). Nicotine (free base), atropine sulfate, choline chloride, and eserine hemisulfate salts are bought from Sigma Chemical Co. (St. Louis, MO, U.S.A.). ACH chloride was supplied by Hoffinann-LaRoche (Basel, Switzerland). Unlabeled acetyl-CoA was purchased from Boehringer Mannheim (Mannheim, F.R.G.). Tetraphenylboron™ (sodium salt) and 3-heptanone was purchased from Aldrich Chemical Co. (Milwaukee, WI, U.S.A.). Ethyl acetate was bought from American Chemicals Co. (Montreal, Quebec, Canada). Bovine serum albumin (98% fatty acid free) and Ecolite scintillation cocktail was purchased from ICN Biochemicals (Irvine, CA, U.S.A.). Triton™ X-100 (100%), scintillation grade, was obtained from Amersham (Arlington, IL, U.S.A.). All other chemicals were obtained from Fisher Scientific (Montreal, Quebec, Canada).

Human brain tissues were obtained at autopsy from individuals clinically diagnosed as having AD, PD, or PD/AD and from neurological normal age-matched controls. Tissues were provided by the Brain Bank of the Douglas Hospital Research Center (Y. Robitaille, neuropathologist). Histopathological criteria have been described earlier. Brain tissue hemispheres to be used for biochemical assays were sectioned into thick (10 mm) coronal slices quickly frozen in 2-methylbutane at -40° C before storage at -80°. For biochemical assays, brain tissue slices were slowly thawed on a cold plate, and the following structures were dissected as follows: frontal (Brodmann areas 9 and 10) and temporal (Brodmann areas 20,21,22, and 38) cortices, hippocampus and cerebellum (used as a low pathology brain area sample).

### iii) Analysis of the results

Binding parameters (Kd and Bmax values) were derived from saturation experiments analyzed by the computerized method LIGAND™ (Aubert I. et al., 1992, *J. Neurochem.,* 58:529-541). Statistical significance of differences between control and AD (0, 1, 2 copies of E4 alleles) brain tissue was evaluated using Student's unpaired t test, with values of p < 0.05 being considered significant.

### iv) Assay for Choline Acetyltransferase (CHAT) Activity

Tissues from various brain regions were homogenized and incubated for 15 min. in buffer containing [14C]acetyl-CoA as previously described in details (Aubert I. et al., 1992, *J. Neurochem.,* 58:529-541) to determine ChAT activity.

Multiple biochemical and anatomopathological studies have shown that the immunoreactivities and activities of cholinergic marker enzymes, such as ChAT, decrease in the neocortex as well as in the hippocampus of patients with AD. As shown in Fig. 6, the apoE4 allele copy number is inversely correlated with ChAT activity in the hippocampus and temporal cortex of age-matched control and AD subjects. In Figs. 6A-J, each bar refers to the mean value + S.E.M. Significant differences between groups are indicated by the number of stars: * = p<0.05; ** = p<0.01; and, *** = p<0.001. Striking reductions in ChAT activity were seen in the brain tissue of apoE4 carriers. ChAT values measured in the hippocampus (23 ADs and 30 controls) and temporal cortex tissue (30 ADs and 12 controls) of AD subjects with different apoE4 allele loads are presented in Figure 4. Statistical analyses indicated that ChAT levels in AD subjects with 1 to 2 copies of the apoE4 allele were significantly lower as compared to AD and control subj ects with 0 to 1 copy of the apoE4 allele.

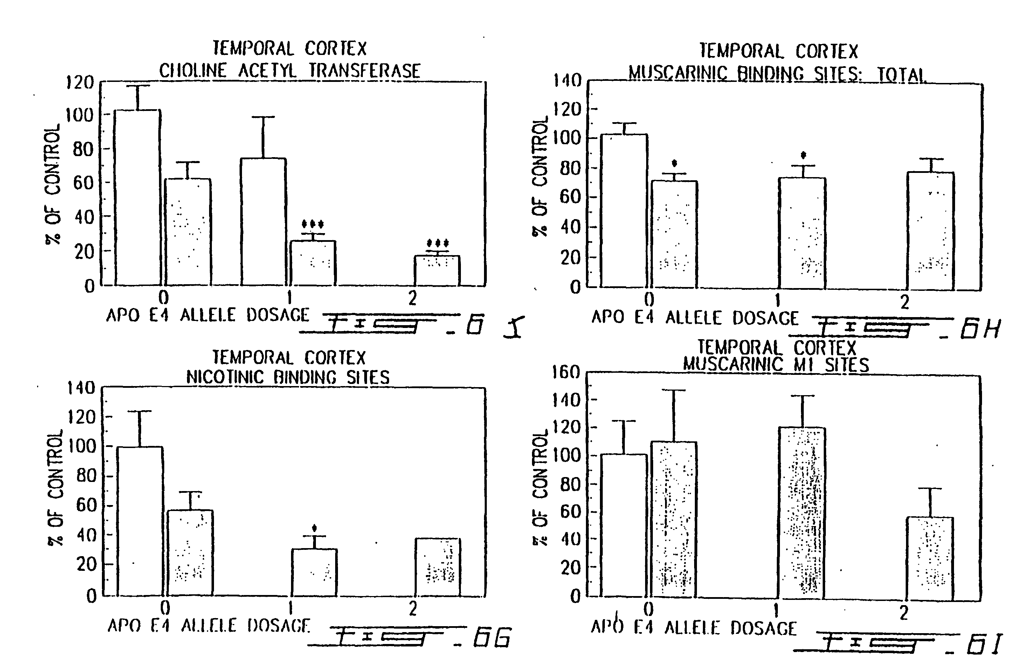

Acetylcholinesterase positive neuron density, in the sub-cortical areas projecting to the temporal cortex and hippocampal structures, are represented in Fig. 7. Neuronal cell density in the Ch2, Ch4a, and Ch4i regions are expressed as a percent of control values. Nucleolar volume is expressed in µm³ and plaque density is expressed as number of plaques per mm² in the hippocampus of the same patients. The genotype of these patients was initially unknown. We have re-analyzed the data for those patients in which genotype could be determined. Three AD subjects were apoE4 negative whereas four AD subjects were apoE4-positive. These results clearly confirm previous findings that AD subjects show marked loss of cholinergic neurons in Ch2, Ch4a, and Ch4i regions, and also highlight the fact that the presence of the apoE4 allele potentiates significant loss of neurons in the Ch2, Ch2a (P<0.01 ), and in the Ch4i (p<0.05) regions.

### v) Binding of [3H]QNB to total populations of muscarinic sites

Punches of cortical and subcortical tissues from control subjects and AD brains were homogenized, centrifuged, and resuspended in Krebs buffer as previously described. Final membrane pellets were suspended in buffer at a concentration of approximately 3-5 mg of protein/ml. Aliquots of membrane-enriched homogenate (0.6-1.0 mg of protein) were incubated in Krebs buffer in the presence of a saturating concentration of [3H]QNB (10 nM) for 60 min. at room temperature (23°C) in a total volume of 0.5 ml. All assays were performed in duplicate. Bound [3H]QNB was separated from free ligand by rapid filtration under reduced pressure through Schleicher & Schuell No. 32 glass filters, presoaked in 0.1% polyethylenimine solution, using a Brandel Cell Harvester apparatus (Brandel, Gaithersburg, MD, U.S.A.). Filters were then rapidly washed with ice-cold buffer, three times with 4.5 ml each, before being dried. The radioactivity of the filters was determined by liquid scintillation counting using a Beckman model LS7000 scintillation counter at 48% efficiency. Nonspecific binding, defined in the presence of 1 µM atropine sulfate, represents usually <15% of total binding.

### vi) Binding of [3H]PZ to human brain muscarinic M1 sites

Brain tissues were processed as previously described herein. Aliquots of final homogenates were incubated in Krebs buffer with various concentrations of [3H]PZ (0.1-20 nM) for 60 min. at room temperature (23°C). Assays were terminated and radioactivity was determined as described above for [3H]QNB binding. Nonspecific binding was defined in the presence of total binding at ligand concentrations approximating Kd values.

### vii) Binding of [3H]AF-DX 116 to human brain muscarinic putative M2 sites

Brain tissues were processed as described above for [3H]QNB assay. Aliquots of final homogenates were incubated in Krebs buffer with various concentrations of [3H]AF-DX 116 (0.1-20 nM) for 60 min. at 4° C. Assays were terminated and radioactivity was determined as described above for [3H]QNB binding. Nonspecific binding was defined in the presence of 1 µM atropine sulfate which represents usually less than 40% of total binding at ligand concentrations approximating Kd values.

Muscarinic binding sites (either total, M1, or M2) were not altered in the hippocampal tissue of AD versus control subjects. The apoE genotype had no significant impact on the activity of these receptors. Muscarinic M1 and M2 receptor sites were not altered in AD versus control subjects in the temporal cortex tissue whereas the so-called total (QNB) muscarinic binding sites were slightly reduced in AD versus control subjects (i.e. no genotypic effect was observed for this receptor group).

### viii) Binding of [3H]MCC to human brain nicotinic sites

Brain tissues were processed as described above for [3H]QNB assay with the exception that samples were homogenized in 50 mM Tris-HCl buffer. Aliquots of final homogenates were incubated in 50 mM Tris-HCl buffer with various concentrations of [3H]MCC (0.1-20 nM) for 60 min. at 4° C. Assays were terminated and radioactivity was determined as described above for [3H]QNB binding assay. Nonspecific binding was defined in the presence of 10 µM nicotine and represents usually less than 50% of total binding at ligand concentrations approximating the Kd values.

### Results

Nicotinic receptor binding sites were significantly reduced in AD subjects carrying an apoE4 allele when compared to AD subjects not carrying an apoE4 allele, which were not different from control subjects. This same correlation was observed for both the hippocampal and the temporal areas in brain tissue taken from AD patients.

These results are consistent with the notion that apoE genotype may directly influence the synaptic plasticity of the cholinergic system in response to neuronal cell loss. In this model, apoE4 compromises lipid homeostasis and consequently impairs membrane remodeling. The fact that the cholinergic system is the only neurotransmitter system of the brain that requires lipid (instead of amino acids) to synthesize its neurotransmitter (the acetylcholine) further highlights the selective vulnerability of this system in a situation of poor lipid delivery and/or availability.

### EXAMPLE V

### Acetylcholinesterase-inhibitor treatment in humans with AD: Clinical effect of apoE genotype

### Study Design

A 30-week study was conducted in which patients were randomized to one of four treatment groups: placebo or escalating doses of the acetylcholine esterase inhibitor tacrine

### Methods

In the tacrine treated group, all patients began treatment at 40 mg/day. One group had tacrine increased to 80 mg/day, a dose on which they remained until the end of the study. The other two groups received tacrine in dosages increased beyond 80 mg/day to 120 and 160 mg/day. Only the latter group of patients was considered in the present genotype analysis.

Forty AD patients who received the maximum dose of tacrine (40 mg/d for 6 weeks, 80 mg/d 6 weeks, 120 mg/d for 6 weeks, and 160 mg/d for 12 weeks for a total of 30 weeks) and completed the drug trial were selected for a determination of their apoE phenotype by assaying serum proteins as previously described (above and Poirier et al., 1993, *Lancet* 342:697-699). Patients were selected on the basis of the presence or absence of a response to the drug treatment. The Alzheimer's Disease Assessment Scale (ADAS) was used to monitor treatment effects. Half of the selected patients showed drug responsiveness (positive AD differences). Patients were then examined for the impact different apoE genotypes (e.g., 2/2, 3/2, 3/3, 3/4, 2/4 and 4/4) had on therapeutic response using the ADAS-cog and the AD total test results obtained before and after tacrine administration. The ADAS-cog is an objective test that evaluates memory, attention span, reasoning, orientation, and praxis: a declining score over time represents a positive difference and indicates improvement (Rosen W.G. et al, 1984, *Am. J. Psychiatr.,* 141:1356 1364). The AD total, includes the cognitive and non-cognitive portion of the ADAS evaluation.

### Results

### Cholinergic Drug Response in Genotyped AD Subjects

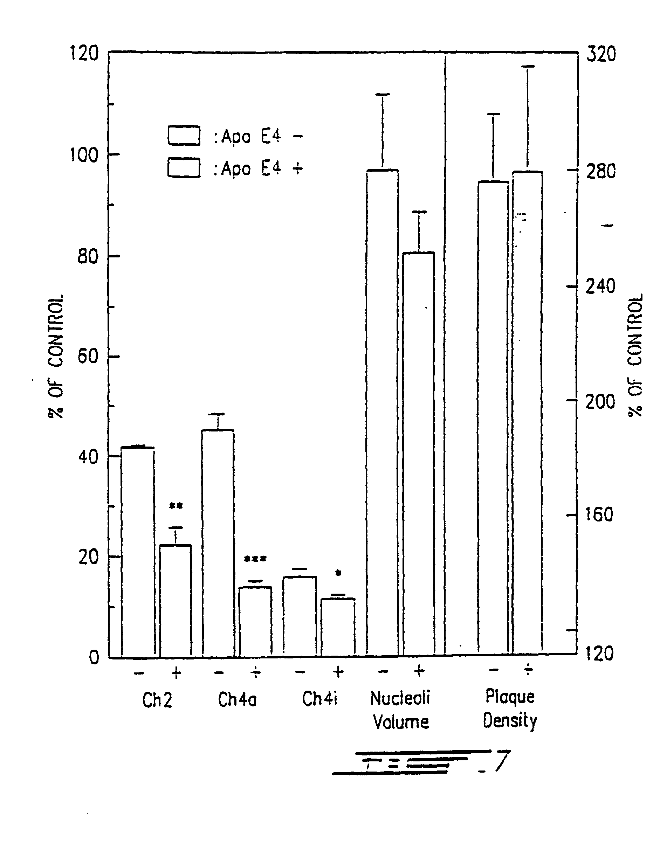

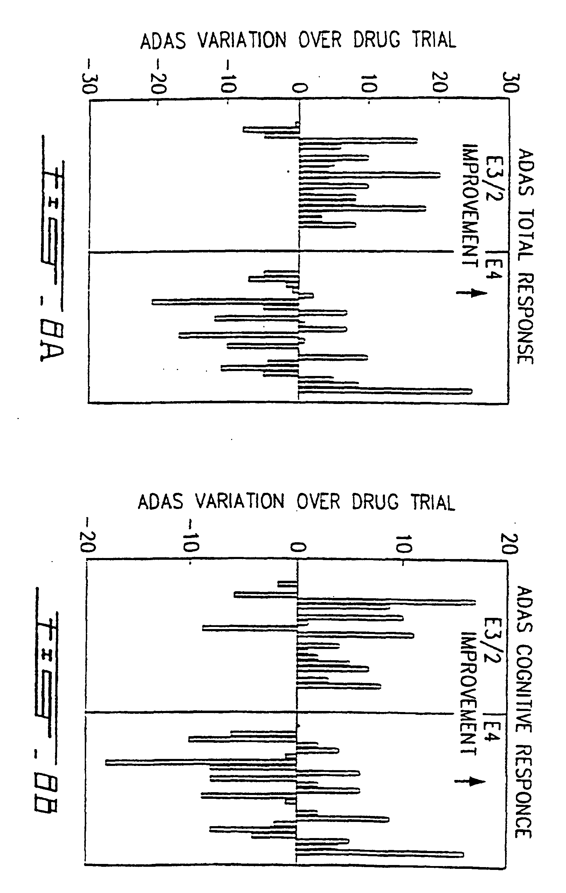

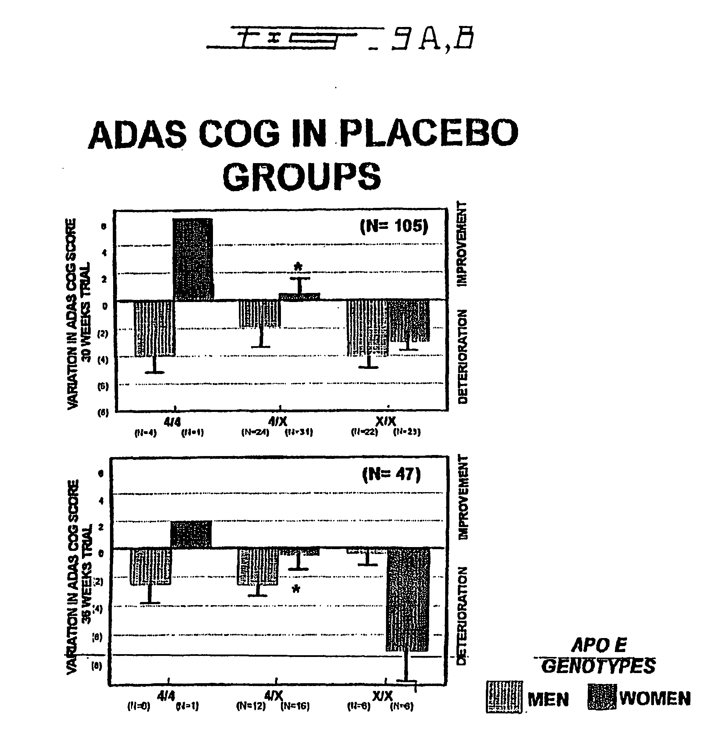

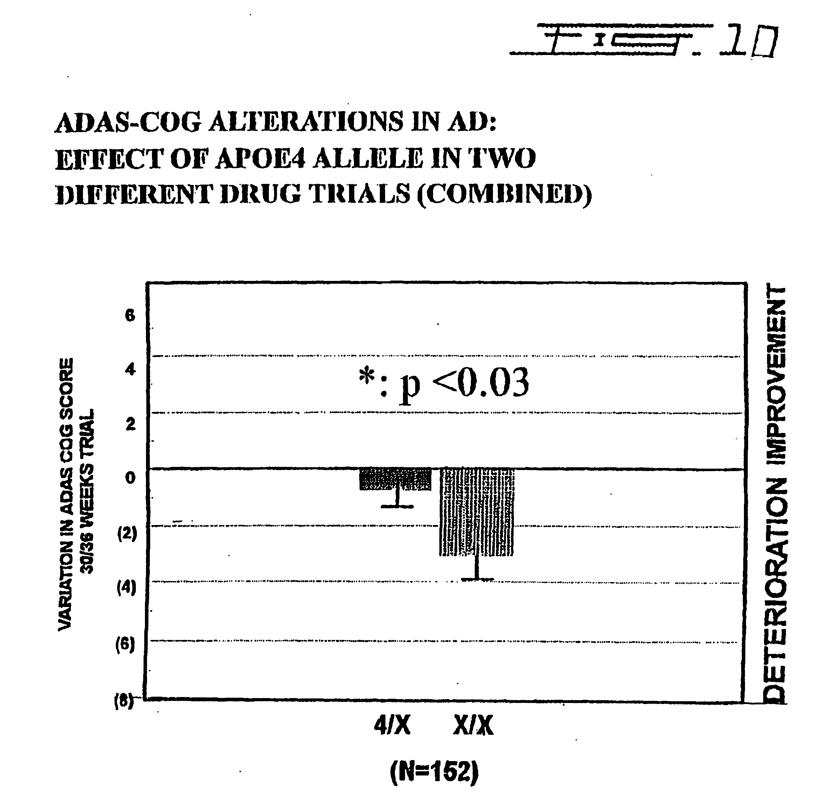

Figure 8 illustrates the drug responsiveness (ADAD-cog and AD total) of AD subjects as a function of the apoE4 allele load. Each bar represents an individual subject. Positive delta values (difference in the AD score at the end of drug treatment minus the AD score prior to drug treatment) indicate improvement in cognitive performance (ADcog) and global performance (ADAS-total). Negative values represent patients who have deteriorated over time during drug treatment. In this study, all the subjects were AD subjects and all were treated with the same drug, at the same level, for the same duration. The only critical factor that distinguished these subjects was the presence or absence of the apoE4 allele.

Our result clearly indicate that more than 85% of the apoE4 negative subjects show improvement with tacrine administration (AD total) whereas 60% of the apoE4 positive subjects experience a decline following drug treatment. In other words, 4 out 5 subjects that do not respond to tacrine are apoE4 positive. The ADAS-cog scale shows a similar response profile in apoE4 carriers and non-carriers.

Taken together, these data clearly suggest that cholinergic function in AD-E3/3, 3/2, and 2/2 subjects were at least partially spared when compared to AD-E4/3, AD-E4/2, and AD-E4/4 carriers. Most importantly, this genetic susceptibility apparently resulted in sub-groups of AD patients which responded differently to cholinomimetic-based therapies; with E4 carriers being at a greater risk for loss of their Ach synthetic capacities. This hypothesis was formally tested in tacrine treated AD subjects that showed different apoE genotypes. As expected, apoE4 negative subjects were found to respond tremendously well to the acetyl cholinesterase inhibitor tacrine (an acetylcholine metabolism enhancer) when compared to apoE4 carriers.

The cholinergic hypothesis of geriatric memory dysfunction (Bartus R.T. et al., 1982, *Science,* 217:408-417) raises some fundamental questions regarding the observed heterogeneity of clinical responses toward various cholinomimetics in different AD patients. The absence of clear beneficial effects of choline and lecithin in geriatric patients with and without AD have always been quite perplexing. Furthermore, clinical trials based on the use of esterase inhibitors such physostigmine and tacrine (Davis K.L. et al, 1992, *N. Engl. J. Med.* 327:1253 1259) have shown that contrary to young subjects, the optimal acute dose necessary to facilitate performance on memory tasks varied considerably among individual aged control subjects and AD patients.

The presence of the apoE4 allele appears now to be the most important factor responsible for individual variations in residual brain cholinergic innervation in AD and a clear predictor of clinical outcome of cholinergic based therapies. Clinical responsiveness to cholinergic agents monitored in genotyped AD patients demonstrated that apoE4 carriers are unlikely to be good responders, at least with the use of Ach precursors and esterase-based therapies.

### EXAMPLE VI

### Effects of sex and apoE genotype on drug and drug dosage efficiency.

In this study we characterized AD patients recruited for a large multi-center study of tacrine, to investigate the impact of apoE allele load on treatment outcome when treated with either a placebo or different doses of tacrine. The patients were classified by sex and dosage. The results are summarized in Table 2.

### Method

### i) Study Design

The 30-week study was a randomized, double-blind, placebo-controlled, and parallel-group clinical trial conducted at 33 centers in the USA (see Knapp, M.J. et al. *JAMA* 271: 985-991 (1995) for complete methodology). Patients were radomized for tacrine treatment received at an initial dose of 40 mg/day (10 mg QID) and were force-titrated at 6-week intervals in increments of 40 mg to maximum dose levels of 80, 120, and 160 mg/day for Groups II, III, and IV, respectively. Group I received a placebo for the entire 30 weeks. The protocol was approved by Institutional Review Boards for each study center and followed guidelines of the Declaration of Helsinki and Good Clinical Practices.

Patients who completed the 30-week study or terminated their participation in the study, were eligible to receive long-term, open-label, tacrine treatment. In the open-label phase, the initial dose for all patients was 40 mg/day (10 mg QID) and could be increased every 4 weeks in 40-mg increments to a maximum dose of 160 mg/day. The protocol specified visits in the open-label phase and visits every 3 months once a patient reached a stable dose. Dosage records were available for all patients. Approximately 2 years after the last patient completed the double-blind phase, the protocol was amended to allow genotyping of baseline plasma samples and collection of information about nursing home placement (NHP) and mortality. Attempts were made, through the study centers, to contact the families of all 663 patients who were originally randomized.

### ii) Patient Population

Patients eligible for the 30-week study were men and women, at least 50 years of age, meeting National Institute of Neurological and Communicative Disorders and Stroke (NINCDS) criteria for a diagnosis of probable AD. Patients were otherwise healthy and did not have significant extra pyramidal, cerebrovascular, cardiac, or hepatic disease; insulin-dependent diabetes mellitus; or chronic renal insufficiency. Disease severity at the time of study entry was defined as mild to moderate based on the Mini-Mental State Examination (MMSE). MMSE scores between 10 and 26 were considered inclusive. Written informed consent was obtained from care givers and patients or their legal representatives. Medications with known central nervous system effects were prohibited during double-blind treatment. Once patients completed or terminated their participation in the double-blind phase of the study, no restrictions on concurrent medications were imposed. Patients who stopped tacrine treatment could take other investigational drugs.

### iii) Outcome Measure

The ADAS-Cog is an objective test that evaluates memory, language, and praxis and is a sensitive measure of patient performance (maximum severity score of 7). A decrease in score indicates improvement.

The CIBIC is a global evaluation of change and is intended to determine whether the effects of an antidementia drug are large enough for detection by an experienced, skilled clinical observer during a comprehensive clinical interview with the patient. At baseline, input from the patient and family members are accepted in addition to a review of the patient's test performance. During subsequent visits, the clinician's assessment is based solely on an interview with the patient, without regard to test performance or family observations. The patient is rated on a 7-point scale: 1, very much better; 4, no change; and 7, very much worse.

The FCCA is also a 7-point scale. Similar to the CIBIC, a review of psychometric test scores is excluded, but input from family members is allowed. The FCCA was conducted after the CIBIC and only when the patient completed or withdrew from the study.

Each site provided data on the dates of death and/or NHP if either occurred during the 2-year follow-up period.

### iv) Statistical Methods

Four comparisons of tacrine 160 mg/day dosage versus a placebo were conducted: (1) without adjusting for apoE4; (2) with adjusting for the presence or absence of apoE4; (3) for patients who carry an apoE4 allele; and, (4) for patients without an apoE4 allele. The estimated treatment differences, 95% confidence intervals for the treatment differences, and parametric p-values were computed from an analysis of variance (ANOVA) that included effects for randomized treatment group (placebo, 80 mg/day, 120 mg/day, and 160 mg/day) and study site. An analysis of covariance (ANCOVA) for ADAS-Cog also included a baseline score as a covariant. Least-square means were used to estimate the effect of the E4 allele on efficacy scores taken at week 30.

Similar ANOVA and ANCOVA models that included effects of the treatment group, apoE4 genotype, site and baseline score (for ADAS-Cog), and treatment and apoE4 interactions were used to test the generalizability of the main effects of treatment in populations with and without the apo E4 allele.

CIBIC and FCCA scores were analyzed using Cochran-Mantel-Haenszel (CMH) methods on modified ridit scores, stratifying by site in the unadjusted analyses and stratifying by site and apoE4 status in the adjusted analyses. Nonparametric p-values for CIBIC and FCCA were used to determine if tacrine treatment effects were statistically significant.

The change in ADAS-Cog scores at Week 30 were estimated for: (1) placebo patients with an apoE4 allele, (2) placebo patients without an apoE4 allele, (3) tacrine 160 mg/day patients with an apoE4 allele, and (4) tacrine 160 mg/day patients without an apoE4 allele using ANCOVA that adjusted for site and baseline score. Similarly, at week 30, CIBIC and FCCA scores were estimated using ANOVA adjusted for study site effects.

PROC LOGISTIC analysis adjusted for baseline Instrumental Activities of Daily Living (IADL), other investigational anti-dementia agents, age, gender, and presence or absence of the apoE4 allele was used for performing logistic regressions to analyze NHP and mortality data. Patients were grouped according to the last daily dose of tacrine received (0-40 mg/day, >40 - 80 mg/day, >80 - 120 mg/day, and >120 - 160 mg/day). Estimated treatment differences between the 0 to 40 mg/day group and 3 other groups were computed using odds ratios. To check for generalizability of the effect of the last daily dose of tacrine, a similar model included additional indicator variables for treatment and apoE4 interactions. NHP and mortality data were also analyzed without adjusting for the presence or absence of the apoE4 allele for all randomized patients and for patients with and without the apoE4 allele.

### Results

Table 2 shows a correlation of drug dosage, patient sex, and apoE4 allele load, and indicates that apoE4 allele load is a critical element in determining an appropriate treatment protocol. For example, men having no apoE4 allele do best with either low or high doses, men with a single apoE4 allele require a high dose for maximum effect, and men having two apoE4 alleles do not respond to tacrine. By contrast, women who have no apoE4 allele do best with a mid-level tacrine dose, while those with one apoE4 allele do equally well at any dose. Like men, those women who lacked an apoE4 allele did not respond to tacrine. When the above data were incorporated into the pharmacogenetic approach, our method allowed one to provide the clinician and the patient with a report providing the lowest dose which indicates the highest effectivity for a given patient. This finding allows for a correlation of the maximum level of therapeutic drug responsiveness with a minimum level of side effects.

**Table 2.**

| ADAS-Cog Improvement in Men and Women Treated with Tacrine and Having Different apoE Allele Loads | | | |
|---|---|---|---|
| MEN | | | |
| | E4/4 | E4/X | EX/X |
| PLACEBO | 0 | 24 | 14 |
| Tacrine low | 0 | 14 | 33 |
| Tacrine mid | 0 | 34 | 0 |
| Tracrine high | - | 69 | 29 |

| WOMEN | | | |
|---|---|---|---|
| | E4/4 | E4/X | EX/X |
| PLACEBO | 0 | 26 | 14 |
| Tacrine low | 0 | 20 | - |
| Tacrine mid | 0 | 24 | 67 |
| Tracrine high | - | 20 | 43 |
| % of subjects with an improvement of the ADAS-COG score by at least 5 points on the scale | | | |

### EXAMPLE VII

### Relationship Between ApoE Genotype and Drug Therapy Outcome in Patients Suffering from Stroke

### Method

We have analyzed 51 patients suffering from stroke to determine if there is a relationship between apoE genotype and drug therapy outcome. Our small study group was composed of Caucasian females, currently diagnosed as suffering from stroke, and currently under treatment with either aspirin or anti-thrombotic drugs (e.g. ticlopidine (Ticlid™). Although our study group is small, and is not randomized by sex, the patients in this study have a apoE allele distribution similar to a much larger randomized North American-population (see Table 3). To determine patient apoE genotypes, 5 mls of whole blood was drawn from each patient and used as a source of genetic material for apoE allele determination as described herein. The patient's apoE genotype was then compared with information from the patient's medical file. We have measured the speed at which patients recover from a stoke incident and the duration of their rehabilitation, in order to assess the potential relationship between a patient's apoE genotype and stroke prognosis.

### Results

**Table 3.**

| apoE Allele Distribution of Study Group Compared to Representative Population | | |
|---|---|---|
| Genotype | % Population (Canada) | Study Group |
| E4/E4 | 3.9% | 0% |
| E4/E3 | 20.6% | 27% |
| E4/E2 | 9.8% | 2% |
| E3/E3 | 61.8% | 59% |
| E3/E2 | 2.0% | 12% |
| E2/E2 | 2.0% | 0% |

In order to determine the relationship between apoE genotype and the time it takes a patient to make a complete recovery from a stroke incident, we have analyzed the data as follows. We have divided the study group into two populations, those patients which recovery quickly on drug therapy (fast responders) and those patients that recovery slowly on drug therapy (slow responders) and asked what is the apoE allele representation (Table 4).

**Table 4.**

| apoE Genotype Distribution in Stroke Patients that Make a Complete Recovery Under Drug Therapy Quickly vs. Slowly | | | | |
|---|---|---|---|---|
| Genotype | % population (Canada) | This Study | Slow Responder | Fast Responder |
| E4/E4 | 3.9% | 0% | 0% | 0% |
| E4/E3 | 20.6% | 27% | *20%* → | *47%* |
| E4/E2 | 9.8% | 2% | 3% | 0% |
| E3/E3 | 61.8% | 59% | 63% ← | 47% |
| E3/E2 | 2.0% | 12% | 14% ← | 7% |
| E2/E2 | 2.0% | 0% | 0% | 0% |

When the genotypes of these two populations were compared, we observed a link between the speed of recovery from stroke and being a carrier of the apoE4 allele. The fastest responders to drug therapy following stroke, were patients carrying the apoE4 allele (Table 4). When these data were analyzed as a function of apoE4 allele load, patients with a greater apoE4 allele load were over represented as making a good recovery (Table 5).

**Table 5.**

| Comparison of apoE4 Genotype Load and Stroke Recovery | | |
|---|---|---|
| Genotype | poor | good |
| +E4 | 19% **→** | 32% |
| - E4 | 81% **←** | 68% |

### Analysis of Age of Onset Versus Response to Drug Therapy

In order to determine the relationship between apoE genotype and age of disease onset, apoE genotype data was analyzed as a function of the age of the patient when the first stroke incident occurred (Table 6). In this study, the majority of the patients, 74%, were non-ApoE4, and the genotypes were well distributed into all age groups, which minimized any favored results due to longevity possibly being linked to apoE genotype.

**Table 6.**

| apoE4 Allele Distribution as a Function of Age When Patient Suffered Stroke Incident | | | | |
|---|---|---|---|---|
| Genotype | 50-60 | 70 | 80 | 90 |
| E4/E4 | 0.0% | 0.0% | 0.0% | 0.0% |
| E4/E3 | 22.2% | 20.0% | 30.8% | 66.7% |
| E4/E2 | 11.1% | 0.0% | 0.0% | 0.0% |
| E3/E3 | 66.7% | 73.3% | 53.8% | 16.7% |
| E3/E2 | 0.0% | 6.7% | 15.4% | 16.7% |
| E2/E2 | 0.0% | 0.0% | 0.0% | 0.0% |

In addition, analysis of this data by age group does not showing major differences regarding treatment efficacy, even though, the group of 70 year old patients seem to globally represent the best response to the disease (Table 7).

**Table 7.**

| Drug Treatment Efficacy for Stroke Patients as a Function of Age | | | | | |
|---|---|---|---|---|---|
| | % Study | 50-60 | 70 | 80 | 90 |
| Poor | 100% | 15% | 31% | 31% | 8% |
| Average | 100% | 18% | 18% | 36% | 14% |
| Good | 100% | 23% | 46% | 8% | 15% |
| Very good | 100% | 0% | 50% | 0% | 0% |

In order to determine if the apoE genotype influences the total time a stroke patient requires for rehabilitation (often a long rehabilitation period is required after a stroke incident), we have analyzed patient rehabilitation time as a function of the patient's apoE genotype. We observed a direct and positive relationship between the presence of an apoE4 allele and a short rehabilitation time (Table 8).

**Table 8.**

| Total Rehabilitation Time Required after Stroke Incident as a Function of Patient apoE Genotype | | | | |
|---|---|---|---|---|
| Genotype | % Population (Canada) | % Study | Quick | Slow |
| E4 | 15.20% | 21% | 28% ← | 19% |
| E3 | 77% | 69% | 64% → | 74% |
| E2 | 7.80% | 10% | 8% | 7% |

In order to assess whether a patient's apoE genotype influences recovery directly after a stroke attack, we have analyzed a patient's ability to make an immediate recovery following a stroke as a function of the patients apoE genotype. We observed that the speed of recovery immediately following a stroke attack is not apoE genotype dependent (Table 9).

**Table 9.**

| Patient Recovery Ability Immediately Following a Stroke as a Function of Patient apoE Genotype | | | | |
|---|---|---|---|---|
| Genotype | % population (Canada) | % Study | Quick | Slow |
| +E4 | 15.2% | 21% | 21% | 25% |
| - E4 | 84.8% | 79% | 79% | 75% |

In summary, we have discovered that a direct link exists between the presence of an apoE4 allele and a stroke patient's outcome. We believe that the apoE4 allele may be beneficial to the stroke patient. We further predict, that the ability to upregulate the expression or stability of the apoE4 gene product, may have beneficial consequences for a patient suffering from stroke. This invention proposes that the ability of a drug to increase apoE4 gene or stability would implicate that drug as a therapeutically-effective drug for aiding recovery from stroke, for example, by reducing rehabilitation time.

### EXAMPLE VIII

### Relationship Between ApoE Genotypes and Drug Therapy Outcome in Patients Suffering from Parkinson's Disease

### Method

We have analyzed 59 patients suffering from Parkinson's disease (PD) to determine if there is a relationship between a patient's apoE genotype and drug therapy outcome. Our small group was composed of Caucasian males, diagnosed as suffering from PD, and currently under treatment with levodopa-carbidopa (Sinemet™). These patients were not suffering from any other central nervous system disease. To determine patient apoE genotypes, 5 mls of whole blood was drawn from each patient and used as a source of genetic material for apoE allele determination as described herein. The patient's apoE genotype was then compared with information from the patient's medical file. Our analysis was based on comparing patient response to treatment, designed to improve symptoms of tremor and rigidity, as a function of the patient's apoE genotype.

### Results

**Table 10.**

| ApoE Genotype Distribution of Study Group, a Larger Population, and Across Different Age Groups | | | | | | |
|---|---|---|---|---|---|---|
| Genotype | % population (Canada) | % Study | 40-50 | 60 | 70 | 80 |
| E4 | 15.20% | 16.3% | 18.8% | 10.7% | 20.0% | 0.0% |
| E3 | 77% | 68.8% | 68.8% | 71.4% | 70.0% | 100.0% |
| E2 | 7.80% | 15.0% | 12.5% | 17.9% | 10.0% | 0.0% |

Although our study group is small, and is not randomized by sex, the patients in this study have an apoE allele distribution similar to a much larger randomized North American-population (Table 10). We also observed a similar distribution among different age groups suggesting that apoE genotype is not linked to longevity (Table 10). We compared the average age of patients who responded well to drug therapy (good responder) versus those patients that did poorly (bad responder), and observed a similar average age, 64-67, that confirmed no age relationship in this data set. An analysis of the apoE allele load between good and bad responders to drug therapy also revealed that age differences did not influence treatment outcome.

In contrast, we observed a strong negative correlation between a patient's response to drug therapy and apoE4 allele load. Patients with no apoE4 allele showed a better response, as measured by improvements in symptoms of rigidity and tremor, then did those patient's with an apoE4 allele (Table 11). Thus, we conclude that a direct link exists between carrying an apoE4 allele and the treatment outcome of a patient with Parkinson's Disease.

**Table 11.**

| Drug Response of Parkinson's Disease Patients with Different apoE Genotypes | | | | |
|---|---|---|---|---|
| Genotype | % population (Canada) | % Study | Bad responder | Good responder |
| E4/E4 | 3.9% | 0% | 0% | 0% |
| E4/E3 | 20.6% | 17% | 20% ← | 8% |
| E4/E2 | 9.8% | 5% | 4% | 4% |
| E3/E3 | 61.8% | 63% | 68% → | 72% |
| E3/E2 | 2.0% | 14% | 4% → | 16% |
| E2/E2 | 2.0% | 2% | 4% | 0% |

| Genotype | % population (Canada) | % Study | Bad responder | Good responder |
|---|---|---|---|---|
| +E4 | 15.2% | 16% | 18% ← | 9% |
| - E4 | 84.8% | 84% | 81% → | 91% |

### EXAMPLE IX

### Relationship Between ApoE Genotype and Drug Therapy Outcome in Patients with Multiple Sclerosis

### Method

We have analyzed 65 patients suffering from Multiple Sclerosis (MS) to determine if there is a relationship between the patient's apoE genotype and drug therapy outcome. Our small group was composed of Caucasian females, diagnosed as suffering from MS, and currently under treatment with interferon β-1B (Betaseron™). To determine patient apoE genotypes, 5 mls of whole blood was drawn from each patient and used as a source of genetic material for apoE allele determination as previously described. The patient's apoE genotype was then compared with information from the patient's medical file. Our analysis was based on comparing patient response to drug treatment of symptomatic exacerbations as a function of the patient's apoE genotype.

### Results

We observed that the patients in this study had an apoE allele distribution similar to a much larger randomized North American-population (Table 12). We also observed a similar distribution among different age groups suggesting that the apoE genotype is not linked to longevity (Table 12).

**Table 12.**

| apoE Genotype Distribution of Study Group, Larger Population, and Amongst Different Age Groups | | | | | |
|---|---|---|---|---|---|
| Genotype | % population (Canada) | % study | <30 | 30-40 | >40 |
| E4 | 15.20% | 15.6% | 9.7% | 21.6% | 12.5% |
| E3 | 77% | 66.7% | 67.7% | 62.2% | 68.8% |
| E2 | 7.80% | 17.8% | 22.6% | 16.2% | 18.8% |

In order to determine the relationship between apoE genotype and patient response to drug therapy designed to lessen the symptomatic flare-ups of MS, we have analyzed the data as follows. We have divided the study group into two populations, those patients which have fewer attacks on drug therapy (good responders) and those patients that have many attacks while on drug therapy (bad responders) and asked what is the apoE allele representation (Table 13). We also report the frequencies of these genotypes in the study group and in a larger population. We have observed that non-apoE4 patients responded better to drug therapy than did those patients that carried an apoE4 (Table 13).

**Table 13.**

| apoE Genotype Distribution in MS Patients Responding Well to Drug Therapy Versus Those Patients Responding Poorly | | | | |
|---|---|---|---|---|
| Genotype | % population (Canada) | % study | Bad responders | Good responders |
| E4/E4 | 3.9% | 0.0% | 0.0% | 0.0% |
| E4/E3 | 20.6% | 15.4% | 8.3% | 8.3% |
| E4/E2 | 9.8% | 6.2% | 6.9% ← | 1.7% |
| E3/E3 | 61.8% | 60.0% | 23.3% → | 33.3% |
| E3/E2 | 2.0% | 16.9% | 5.0% → | 13.3% |
| E2/E2 | 2.0% | 1.5% | 1.7% | 0.0% |

When we analyzed these patients by apoE4 allele presence, the correlation between those patients that respond well and the lack of an apoE4 allele is even more striking (Table 14).

**Table 14.**

| apoE4 Allele Load in MS Patients Responding Well to Drug Therapy Versus Those Patients Responding Poorly | | | | |
|---|---|---|---|---|
| Genotype | % population (Canada) | % study | Bad | Good |
| +E4 | 15.2% | 16.5% | 6.7% ← | 5.6% |
| - E4 | 84.8% | 65.9% | 20.0% → | 43.3% |

In another analysis, we studied the correlation between apoE4 allele load and the qualitative nature of the MS attacks each year while undergoing drug treatment. The following results are subdivide into 8 groups, mild attacks reacting badly to their treatment compared with mild attacks having a good response to treatment, and severe attacks compared the same way (Table 15). We observed a distinct impact of the apoE allele load and the efficacy of the drug treatment of MS when the diminution in the number of worsening attacks was measured (Table 15).

**Table 15.**

| apoE4 Allele Load in MS Patients with a Qualitative Change in Attacks | | | | |
|---|---|---|---|---|
| Genotype | mild-bad | mild-good | severe-bad | severe-good |
| +E4 | 3% | 2% | 5% | 7% |
| - E4 | 15% **→** | 34% | 14% **→** | 20% |

It is clear, from these results, that the Apolipoprotein E gene is not only linked to the drug efficacy in Alzheimer's disease, but also directly related to drug efficacy in different CNS diseases such as stroke, Parkinson's disease, and Multiple Sclerosis. Due to the specific role of the apoE gene and its implication in cell regeneration and plasticity requirements specific to a disease, it is expected that the disease relevant genotype will vary from disease to disease.

### EXAMPLE X

### Determination of Risk Prognosis and Therapeutic Treatment Outcome of Stroke Events Using Genetic Analysis of apoE4 and BCHE-K

Stroke is an acute neurologic event leading to death of neural tissue of the brain and resulting in loss of motor, sensory, and/or cognitive function. It is said to be the third leading cause of death in the United States. Genetic predisposition may be important in the pathogenesis of stroke. Such predisposition may not only include genes contributing to elevated blood pressure but also genes acting independently of blood pressure. Twins studies and familial aggregation support evidence for genetic factors contributing to stroke with a polygenic aetiology.

Genetic factors contributing to stroke pathogenesis are poorly established. The following genes and polymorphisms have been implicated in stroke: ACE insertion/deletion polymorphism, factor V gene, factor VII gene, PIA2 polymorphism of the glycoprotein IIIa gene, ApoE, and the interleukin 1β converting enzyme (ICE) gene family.

Butyrylcholinesterase (BChE) is expressed in most human tissues, but its precise metabolic function in the body is still unknown. The polymorphic gene variant BCHE-K, consisting of a point mutation at nucleotide 1615 (GCA to ACA) which changes alanine 539 to threonine, has reduced catalytic activity (Bartels et al., *Am. J. Hum. Genet.* 50:1086-1103, 1992). Recent research from Lehmann et al. *(Hum. Mol. Genet.* 11:1933-1936 (1997)) suggests that BCHE-K is associated with a further increase in the risk of late-onset AD in apoE4 carriers.

We have discovered that the combination of ApoE4 and BCHE-K contribute to define an individual's risk for the development of stroke. We have determined the ApoE4 and BCHE-K genotype for 50 female stroke patients and 64 age and sex matched healthy controls (Table 16). In the control group, we observed 17 out of 64 subjects were heterozygote and 4 out of 64 subjects were homozygote carriers of the BCHE-K allele. We observed that 15 out of 64 subjects carried one copy of the apoE4 allele. Both of these allele distributions fit a Hardy-Weinberg equilibrium for the identified allele frequency (Table 16). In the stroke patients group, 22 out of 50 subjects carried one copy of BCHE-K, and 3 out of 50 subjects were observed to carry two BCHE-K alleles. Of these 50 subjects, 15 were carriers of one apoE4 allele. The identified carrier status was in agreement with a Hardy-Weinberg population for the observed allele frequency (Table 16).

**Table 16.**

| Allelic Frequencies of apoE4 and BCHE-K | | | | | |
|---|---|---|---|---|---|
| | No. of subjects | F:M ratio | mean age | AF BCHE-K | AF ApoE4 |
| controls >58 years | 64 | F only | 72 | 0.195 | 0.117 |
| Stroke cases | 50 | F only | | 0.28 | 0.15 |
| p=(X-square, Yates corr.) | | | | 0.18 (NS) | 0.6 (NS) |

For subjects 58 years of age and older, the allelic frequency of BCHE-K was 0.195 in controls and 0.28 in the 50 stroke cases, providing an odds ratio of stroke of 1.6 (based on allele frequencies) and 2.1 (based on carrier frequencies) (Table 17.). In apoE4 carriers, the odds ratio of stroke was about 1 (Table 17).

**Table 17.**

| Odds Ratios of Stroke for BCHE-K Alleles | | | | | | |
|---|---|---|---|---|---|---|
| subjects | controls | cases | Odds ratio (alleles) | 95% Cl | Odds ratio (carriers) | 95% Cl |
| all | 64 | 50 | 1.6 | 0.9-2.9 | 2.1 | 1.0-4.4 |
| apoE4 carriers | 15 | 15 | | | 1.3 | 0.3-5.6 |

The allelic frequency of apoE4 was 0.12 in controls and 0.15 in 50 stroke cases, giving an odds ratio of stroke of 1.3 (based on allele frequencies) and 1.4 (based on carrier frequencies) (Table 18). In BCHE-K carriers, the odds ratio of stroke was 1.9 (calculated for carrier status) (Table 18).

**Table 18.**

| Odds Ratios of Stroke for apoE4 Alleles | | | | | | |
|---|---|---|---|---|---|---|
| subjects | controls | cases | Odds ratio (alleles) | 95% Cl | Odds ratio (carriers) | 95% Cl |
| all | 64 | 50 | 1.3 | 0.6-2.8 | 1.4 | 0.6-3.2 |
| BCHE-K carriers | 21 | 25 | | | 1.9 | 0.5-7.2 |

Taking account of the carrier status of both gene mutations, we have discovered that there is a two fold increase (8% vs 18%) in stroke cases, compared to controls, in carriers with both the apoE4 and BCHE-K allele (Table 19). This trend is also seen in the apoE4 carriers.

**Table 19.**

| Proportions of Controls and Stroke with Both BCHE-K and apoE4 | | | |
|---|---|---|---|
| subjects | Controls | Stroke cases | P=(X-square, Yates corr.) |
| all | 5/64(8%) | 9/50(18%) | 0.17 (NS) |
| apoE4 carriers | 5/15 (33%) | 9/15 **(60%)** | 0.27 (NS) |

In Table 20, we provide the odds ratio of stroke for subjects carrying at least one allele of apoE4 and BCHE-K as compared to control subjects who have neither allele. In female subjects over 58 years of age who carry both the apoE4 and BCHE-K alleles, the odds ratio of sustaining a stroke was 2.8 fold higher than age matched controls. These data predict for female carriers of this genetic status, an almost 3 fold higher risk for stroke.

**Table 20.**

| Odds Ratio of Stroke | | | | | |
|---|---|---|---|---|---|
| apoE4 | BCHE-K | controls | Stroke | Odds ratio (carriers) | 95% Cl |
| no | no | 33 | 21 | Reference | |
| yes | yes | 5 | 9 | 2.8 | 0.9-9.2 |

In summary, we have discovered that determining an individuals's apoE4 and BCHE-K allele status is a useful tool in the prediction of an individual's risk for stroke. Furthermore, our results demonstrate that prognostic forecasting could allow patients to start prophylactic therapies before disease strikes. For example, the risk of stroke could be calculated for asymptomatic and healthy individuals as young adults and well before a stroke incident has occurred. Then as the individual ages, preventive therapies could be invoked in order to prevent or lessen the likelihood of a catastrophic stroke incident later in life.

## Claims

1. A method of creating a prognosis protocol for a patient diagnosed with neurofibromatosis, a pathology of the developing nervous system, depression, a nervous system injury, an infection of the nervous system, coma, multi-infarct dementia, a dietary deficiency, or a cardiovascular injury, said method comprising:
a) identifying a patient already diagnosed with said disease;
b) determining the *apoE* allele load of said patient by genotyping or phenotyping, said phenotyping including characterizing an ApoE protein isoform; and
c) converting the data obtained from step b) into a prognosis protocol, said prognosis protocol including a prediction of drug efficacy and patient outcome, wherein the presence of one or more apoE4 alleles in said patient is indicative of said patient being unlikely to benefit from cholinomimetic therapy.

2. A method of creating a prognosis protocol for a patient diagnosed with stroke, said method comprising:
a) identifying a patient already diagnosed with stroke;
b) determining the *apoE* allele load of said patient by genotyping or phenotyping, said phenotyping including characterizing an ApoE protein isoform; and
c) converting the data obtained from step b) into a prognosis protocol, said prognosis protocol including a prediction of drug efficacy and patient outcome, wherein the presence of one or more apoE4 alleles in said patient is indicative of said patient being likely to benefit from cholinomimetic therapy.

3. The method of claim 1 or 2, wherein said method further comprises obtaining data pertaining to the patient for whom the prognosis protocol is created, wherein said data include information on the patient's diagnosis, age, sex, and genotype, wherein said genotype is the presenilin genotype or the apolipoprotein C1 genotype.

4. The method of claim 1, wherein said pathology of the developing nervous system is fragile X syndrome or a congenital defect in amino acid metabolism.

5. The method of claim 4, wherein said congenital defect is seleded from the group consisting of argininosuccinicaciduria, cystathioninuria, histidinemia, homocystinuria, hyperammonemia, phenylketonuria, and tyrosinemia.

6. A method for identifying patients suitable for participation in a clinical trial of a drug for the treatment of a disease selected from the group consisting of neurofibromatosis, a pathology of the developing brain, depression, a brain injury, an infection of the brain, coma, multi-infarct dementia, a dietary deficiency, or a cardiovascular injury, said method comprising:
a) identifying a patient with said disease;
b) determining the *apoE* allele load of said patient by genotyping or phenotyping, said phenotyping including characterizing an ApoE protein isoform; and
c) converting the data obtained from step b) into a prognosis protocol, said prognosis protocol indicating whether or not said patient is a candidate for a cholinomimetic drug trial or non-cholinomimetic drug trial, wherein a patient lacking both apoE4 alleles is expected to be a candidate in a cholinomimetic drug trial or wherein a patient having one or more apoE4 alleles is expected to be a poor candidate in a cholinomimetic drug trial

7. The method of claim 3, wherein said method further comprises determining the BChE genotype of said patient.

8. The method of claim 1, wherein the presence of one or more apoE4 alleles in said patient is indicative of said patient having a worse prognosis of recovery.

9. The method of claim 2, wherein the presence of one or more apoE4 alleles in said patient is indicative of said patient having a better prognosis of recovery.

10. A method for determining the risk of stroke in a patient, said method comprising:
a) determining the *apoE* allele load of said patient by genotyping or phenotyping, said phenotyping including characterizing an ApoE protein isoform; and
b) determining the BChE genotype of said patient, wherein the presence of one or more apoE4 alleles and one or more BChE-K alleles in said patient indicates said patient is at risk of stroke.

11. A method for performing pharmacogenetic analysis, said method including a means for converting a patient profile into a prognosis protocol, wherein said means includes determining the *apoE* allele load of a patient diagnosed with neurofibromatosis, a pathology of the developing nervous system, depression, a nervous system injury, an infection of the nervous system, coma, multi-infarct dementia, a dietary deficiency, a cardiovascular injury, or stroke by genotyping or phenotyping, said phenotyping including characterizing an ApoE protein isoform.

12. The method of claim 11, wherein said method contains a means for performing the steps of said conversion.

13. The method of claim 11, wherein said method contains a means for compiling the data for said patient profile.

## Patentansprüche

1. Verfahren zur Erstellung eines Prognoseprotokolls für einen Patienten, bei dem Neurofibromatose, eine Erkrankung des entwickelnden Nervensystems, Depression, eine Verletzung des Nervensystems, eine Infektion des Nervensystems, Koma, Multiinfarkt-Demenz, ein Diät-Defizit oder ein kardiovaskulärer Schaden diagnostiziert wurde, wobei das Verfahren umfasst:
a) Identifizierung eines Patienten, bei dem die Erkrankung bereits diagnostiziert wurde;
b) Bestimmung des apoE Allel -Gehalts des Patienten durch Genotypisierung oder Phenotypisierung, wobei diese Phenotypisierung die Charakterisierung einer ApoE Proteinisoform einschließt; und
c) Konvertieren der Daten, die aus Schritt b) erhalten wurden in ein Prognoseprotokoll, wobei das Prognoseprotokoll eine Vorhersage der Arzneimittelwirksamkeit und des Patientenergebnisses einschließt, wobei die Anwesenheit von einem oder mehreren apoE4 Allelen in dem Patienten darauf hinweist, dass der Patient wahrscheinlich nicht von cholinomimetischer Therapie profitieren wird.

2. Verfahren zur Erstellung eines Prognoseprotokolls für einen Patienten, bei dem Schlaganfall diagnostiziert wurde, wobei dass Verfahren umfasst:
a) Identifizierung eines Patienten, bei dem Schlaganfall bereits diagnostiziert wurde;
b) Bestimmung des apoE Allel -Gehalts des Patienten durch Genotypisierung oder Phenotypisierung, wobei diese Phenotypisierung die Charakterisierung einer ApoE Proteinisoform einschließt; und
c) Konvertieren der Daten, die aus Schritt b) erhalten wurden in ein Prognoseprotokoll, wobei das Prognoseprotokoll eine Vorhersage der Arzneimittelwirksamkeit und des Patientenergebnisses einschließt, wobei die Anwesenheit von einem oder mehreren apoE4 Allelen in dem Patienten darauf hinweist, dass der Patient wahrscheinlich von cholinomimetischer Therapie profitieren wird.

3. Verfahren gemäß Anspruch 1 oder 2, wobei das Verfahren weiterhin das Erhalten von Daten einschließt, die den Patienten betreffen, von dem das Prognoseprotokoll erstellt wird, wobei diese Daten Informationen hinsichtlich der Diagnose des Patienten, des Alters, des Geschlechts und des Genotyps einschließen, wobei der Genotyp der Presenilin-Genotyp oder der Apolipoprotein C1 Genotyp ist.

4. Verfahren gemäß Anspruch 1, wobei die Erkrankung des entwickelnden Nervensystems das Fragile X Syndrom oder ein kongenitaler Defekt im Aminosäuremetabolismus ist.

5. Verfahren gemäß Anspruch 4, wobei der kongenitale Defekt ausgewählt ist aus der Gruppe bestehend aus Argininosukzinoazidurie, Cystathioninurie, Histidinämie, Homocystinurie, Hyperammonämie, Phenylketonurie und Tyrosinämie.

6. Verfahren zur Identifizierung von Patienten, die geeignet sind zur Teilnahme an einer klinischen Studie eines Arzneimittels zu Behandlung einer Erkrankung, ausgewählt aus der Gruppe bestehend aus Neurofibromatose, einer Erkrankung des entwickelnden Gehirns, Depression, einer Verletzung des Gehirns, einer Infektion des Gehirns, Koma, Multiinfarkt-Demenz, einem Diät-Defizit oder einem kardiovaskulären Schaden, wobei das Verfahren umfasst:
a) Identifizierung eines Patienten mit der Erkrankung;
b) Bestimmung des apoE Allel Gehalts des Patienten durch Genotypisierung oder Phenotypisierung, wobei diese Phenotypisierung die Charakterisierung einer ApoE Proteinisoform einschließt; und
c) Konvertieren der Daten, die aus Schritt b) erhalten wurden in ein Prognoseprotokoll, wobei das Prognoseprotokoll darauf hinweist, ob der Patient ein Kandidat für eine cholinomimetische Arzneimittelstudie oder eine nicht-cholinomimetische Arzneimittelstudie ist, oder nicht, wobei erwartet wird, dass ein Patient, dem beide ApoE Allele fehlen, ein Kandidat für eine cholinomimetische Arzneimittelstudie ist oder wobei erwartet wird, dass ein Patient, der ein oder mehrere apoE4 Allele hat, ein schlechter Kandidat in einer cholinomimetischen Arzneimittelstudie ist.

7. Verfahren gemäß Anspruch 3, wobei dieses Verfahren weiterhin die Bestimmung des BChE Genotyps des Patienten umfasst.

8. Verfahren gemäß Anspruch 1, wobei die Anwesenheit von einem oder mehreren apoE4 Allelen in dem Patienten darauf hinweist, dass der Patient eine schlechtere Genesungsprognose hat.

9. Verfahren gemäß Anspruch 2, wobei die Anwesenheit von einem oder mehreren apoE4 Allelen in dem Patienten darauf hinweist, dass der Patient eine bessere Genesungsprognose hat.

10. Verfahren zur Bestimmung des Risikos eines Schlaganfalls in einem Patienten, wobei das Verfahren umfasst:
a) Bestimmung des *apoE* Allel Gehalts des Patienten durch Genotypisierung oder Phenotypisierung, wobei diese Phenotypisierung die Charakterisierung einer ApoE Proteinisoform einschließt; und
b) Bestimmung des BChE Genotyps des Patienten, wobei die Anwesenheit von einem oder mehreren apoE4 Allelen und einem oder mehreren BChE-K Allelen in dem Patienten darauf hinweist, dass der Patient gefährdet für Schlaganfall ist.

11. Verfahren zur Durchführung einer pharmakogenetischen Analyse, wobei das Verfahren ein Mittel zur Konvertierung eines Patientenprofils in ein Prognoseprotokoll einschließt, wobei dieses Mittel die Bestimmung des *apoE* Allel Gehalts eines Patienten einschließt, bei dem Neurofibromatose, eine Erkrankung des entwickelnden Nervensystems, Depression, eine Verletzung des Nervensystems, eine Infektion des Nervensystems, Koma, Multiinfarkt-Demenz, ein Diät-Defizit, ein kardiovaskulärer Schaden oder Schlaganfall diagnostiziert wurde, durch Genotypisierung oder Phenotypisierung, einschließlich der Charakterisierung einer ApoE Proteinisoform.

12. Verfahren gemäß Anspruch 11, wobei das Verfahren ein Mittel zur Durchführung der Schritte der Konvertierung enthält.

13. Verfahren gemäß Anspruch 11, wobei das Verfahren ein Mittel zur Erstellung der Daten von dem Patientenprofil enthält.

## Revendications

1. Procédé pour la création d'un protocole de pronostic pour un patient diagnostiqué avec une neurofibromatose, une pathologie du système nerveux en développement, une dépression, une lésion du système nerveux, une infection du système nerveux, un coma, une démence plurilacunaire, une carence alimentaire, ou une lésion cardio-vasculaire, ledit procédé comprenant:
a) l'identification d'un patient déjà diagnostiqué avec ladite affection;
b) la détermination de la charge d'allèle *apoE* du dit patient par génotypage ou phénotypage, ledit phénotypage incluant la caractérisation d'une isoforme de protéine ApoE; et
c) la conversion des données obtenues à partir de l'étape b) en un protocole de pronostic, ledit protocole de pronostic comportant une prédiction de l'efficacité d'un médicament et de l'évolution du patient, tandis que la présence d'un ou plusieurs allèles apoE4 chez ledit patient est indicative de ce qu'il est peu probable que ledit patient tire bénéfice d'une thérapie cholinomimétique.

2. Procédé pour la création d'un protocole de pronostic pour un patient diagnostiqué avec un ictus, ledit procédé comprenant:
a) l'identification d'un patient déjà diagnostiqué avec un ictus;
b) la détermination de la charge d'allèle *apoE* du dit patient par génotypage ou phénotypage, ledit phénotypage incluant la caractérisation d'une isoforme de protéine ApoE; et
c) la conversion des données obtenues à partir de l'étape b) en un protocole de pronostic, ledit protocole de pronostic comportant une prédiction de l'efficacité d'un médicament et de l'évolution du patient, tandis que la présence d'un ou plusieurs allèles apoE4 chez ledit patient est indicative de ce qu'il est probable que ledit patient tire bénéfice d'une thérapie cholinomimétique.

3. Procédé selon la revendication 1 ou 2, dans lequel ledit procédé comprend en outre l'obtention de données concernant le patient pour lequel le protocole de pronostic est créé, tandis que lesdites données comportent des informations sur le diagnostic, l'âge, le sexe, et le génotype du patient, ledit génotype étant le génotype de préséniline ou le génotype d'apolipoprotéine C1.

4. Procédé selon la revendication 1, dans lequel ladite pathologie du système nerveux en développement est le syndrome de fragilité du chromosome X ou une anomalie congénitale dans le métabolisme des acides aminés.

5. Procédé selon la revendication 4, dans lequel ladite anomalie congénitale est choisie dans le groupe consistant en l'acidurie argininosuccinique, la cystathioninurie, l'histidinémie, l'homocystinurie, l'hyperammoniémie, la phénylcétonurie et la tyrosinémie.

6. Procédé pour l'identification de patients convenant pour une participation à un essai clinique d'un médicament pour le traitement d'une affection choisie dans le groupe consistant en une neurofibromatose, une pathologie du cerveau en développement, une dépression, une lésion du cérébrale, une infection du cerveau, un coma, une démence plurilacunaire, une carence alimentaire, ou une lésion cardio-vasculaire, ledit procédé comprenant:
a) l'identification d'un patient ayant ladite affection;
b) la détermination de la charge d'allèle *apoE* du dit patient par génotypage ou phénotypage, ledit phénotypage incluant la caractérisation d'une isoforme de protéine ApoE; et
c) la conversion des données obtenues à partir de l'étape b) en un protocole de pronostic, ledit protocole de pronostic indiquant si ledit patient est ou non un candidat pour un essai de médicament cholinomimétique ou un essai de médicament non-cholinomimétique, tandis qu'on s'attend à ce qu'un patient dénué des allèles apoE4 soit un candidat dans un essai de médicament cholinomimétique ou tandis qu'on s'attend à ce qu'un patient ayant l'un ou plusieurs allèles apoE4 soit un mauvais candidat dans un essai de médicament cholinomimétique.

7. Procédé selon la revendication 3, dans lequel ledit procédé comprend en outre la détermination du génotype BChE du dit patient.

8. Procédé selon la revendication 1, dans lequel la présence d'un ou plusieurs allèles apoE4 chez ledit patient est indicative de ce que ledit patient a un mauvais pronostic de rétablissement.

9. Procédé selon la revendication 2, dans lequel la présence d'un ou plusieurs allèles apoE4 chez ledit patient est indicative de ce que ledit patient a un meilleur pronostic de rétablissement.

10. Procédé pour la détermination du risque d'ictus chez un patient, ledit procédé comprenant:
a) la détermination de la charge d'allèle *apoE* du dit patient par génotypage ou phénotypage, ledit phénotypage incluant la caractérisation d'une isoforme de protéine ApoE; et
c) la détermination du génotype BChE du dit patient, tandis que la présence d'un ou plusieurs allèles apoE4 et d'un ou plusieurs allèles BChE-K chez ledit patient indique que ledit patient court un risque d'ictus.

11. Procédé pour la réalisation d'analyse pharmacogénétique, ledit procédé comportant un moyen pour la conversion d'un profil de patient en un protocole de pronostic, tandis que ledit moyen comporte la détermination de la charge d'allèle apoE chez un patient diagnostiqué avec une neurofibromatose, une pathologie du système nerveux en développement, une dépression, une lésion du système nerveux, une infection du système nerveux, un coma, une démence plurilacunaire, une carence alimentaire, une lésion cardio-vasculaire ou un ictus par génotypage ou phénotypage, ledit phénotypage comportant la caractérisation d'une isoforme de protéine ApoE.

12. Procédé selon la revendication 11, dans lequel ledit procédé contient un moyen pour la réalisation des étapes de ladite conversion.

13. Procédé selon la revendication 11, dans lequel ledit procédé contient un moyen pour la compilation des données pour ledit profil de patient.
